# EUROPEAN PATENT SPECIFICATION

(11) **EP 4 693 247 B1**
(45) Date of publication and mention of the grant of the patent: **04.11.2026**
(21) Application number: 24832295.0
(22) Date of filing: 03.06.2024
(51) Int. Cl.: G08B 21/04, G08B 25/10, G08B 25/00, G01P 15/00, A61B 5/024, A61B 5/01, G06F 1/16, H04M 1/72418

(54) **WEARABLE DEVICE AND METHOD FOR IDENTIFYING TAPPING PATTERN**
TRAGBARE VORRICHTUNG UND VERFAHREN ZUR IDENTIFIZIERUNG EINES ABZAPFMUSTERS
DISPOSITIF POUVANT ÊTRE PORTÉ ET PROCÉDÉ D'IDENTIFICATION D'UN MOTIF DE TAPOTEMENT

(30) Priority: 30.06.2023 KR 20230085390; 25.07.2023 KR 20230097065
(43) Date of publication of application: 11.02.2026
(73) Proprietor: Samsung Electronics Co., Ltd., Suwon-si, Gyeonggi-do 16677 (KR)
(72) Inventor: LEE, Kwangjo, Suwon-si Gyeonggi-do 16677 (KR); CHO, Daesung, Suwon-si Gyeonggi-do 16677 (KR); JOO, Suntae, Suwon-si Gyeonggi-do 16677 (KR)
(74) Representative: HGF
(86) International application number: PCT/KR2024/007591
(87) International publication number: WO 2025/005514

(56) References cited:
- KR-A- 20160 015 050
- KR-A- 20180 023 942
- KR-A- 20180 068 000
- KR-A- 20190 049 336
- KR-A- 20210 057 353
- US-A1- 2020 241 641

## Description

### [Technical Field]

The following descriptions relate to a wearable device and a method for identifying a tapping pattern.

### [Background Art]

A wearable device may be used in a state of being worn by a part of a body of a user. The wearable device may be provided as various forms of products. For example, the wearable device may include a ring shaped device for the user to be worn by the part of the body of the user. The wearable device may provide various user experiences to the user by including a sensor for providing user-associated information.

The above-described information may be provided as a related art for a purpose of helping understanding of the present disclosure. No claim or determination is raised as to whether any of the above-described descriptions may be applied as a prior art related to the present disclosure.
Document US 2020/241641 A1 discloses, according to its abstract, that a user controls an external electronic device with wireless ring device; the ring is disposed on one or more fingers of a hand of the user. The ring detects, via a touch-sensitive input device, an input. In response to detecting the input, in accordance with a determination that the input corresponds to a respective touch event of a plurality of touch events, the ring transmits, to the external electronic device, a command associated with the respective touch event, and generates, via the one or more tactile output generators, a first tactile output indicative of the transmission of the command.
Documents KR 2016 0015050 A and US 2016/034742 A1 disclose, according to their abstracts, a ring-type terminal including a main body configured to be placed on and surround a user's finger and including an insertion region in which the finger is inserted; a fingerprint sensor provided on at least one region of an inner circumferential surface of the main body and configured to recognize a fingerprint of the finger; a guide module provided on the inner circumferential surface such that the finger comes in contact with the fingerprint sensing module while the finger is inserted in the insertion region; and a controller configured to execute a function based on the fingerprint sensed by the fingerprint sensing module.

### [Disclosure]

### [Technical Solution]

The scope of protection conferred is determined from the claims.

### [Description of the Drawings]

FIG. 1 is a block diagram of an electronic device within a network environment according to an embodiment.
FIG. 2 illustrates an example of a wearable device and external electronic devices connected with the wearable device, according to an embodiment.
FIG. 3A illustrates an example of a simplified block diagram of a wearable device according to an embodiment.
FIG. 3B is an example of a partial cross-sectional view of a wearable device according to an embodiment.
FIG. 4 illustrates a flowchart of an operation of a wearable device according to an embodiment.
FIGS. 5A to 5D illustrate an example of data identified through an acceleration sensor according to an embodiment.
FIGS. 6A to 6D illustrate an example of a plurality of tapping patterns according to an embodiment.
FIG. 7 illustrates an example of an operation of a wearable device for identifying an impact pattern according to an embodiment.
FIG. 8 illustrates a flowchart of an operation of a wearable device according to an embodiment.
FIG. 9 illustrates a flowchart of an operation of a wearable device according to an embodiment.
FIG. 10A illustrates a flowchart of an operation of a wearable device according to an embodiment.
FIG. 10B illustrates an example of an operation of a wearable device according to an embodiment.
FIG. 11 illustrates a flowchart of an operation of a wearable device according to an embodiment.
FIG. 12 illustrates a flowchart of an operation of a wearable device according to an embodiment.
FIG. 13 illustrates an example of an operation of training a model in a wearable device according to an embodiment.

### [Mode for Invention]

Hereinafter, an embodiment of the present disclosure will be described in detail with reference to a drawing so that those having ordinary knowledge in the art to which the present disclosure belongs may easily implement the present disclosure. However, the present disclosure may be implemented in various different forms and is not limited to the embodiment described herein. In association with a description of the drawing, the same or similar reference numerals may be used for the same or similar components. In addition, in the drawing and the associated description, a description of a well-known function and a configuration may be omitted for clarity and brevity.

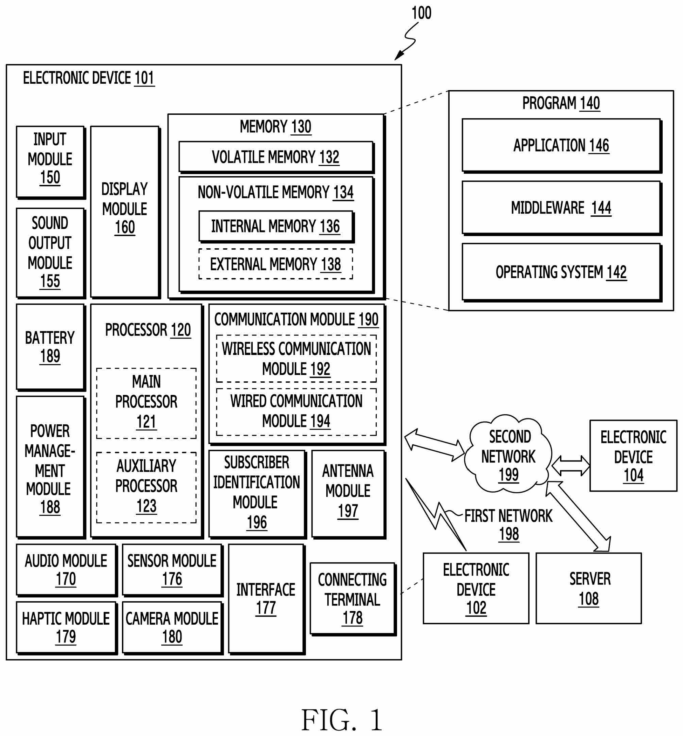

FIG. 1 is a block diagram illustrating an electronic device 101 in a network environment 100 according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an **HDMI** connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101.

The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

According to an embodiment, a wearable device (e.g., the electronic device 101) may operate in a state of being worn by the user. The wearable device may identify (or detect) a physical activity of the user in the state of being worn by the user. For example, the wearable device may identify movement of a part of a body of the user in a state of being worn on the part (e.g., a finger) of the body of the user. The user may perform a function corresponding to the movement by moving the part of the body of the user.

For example, the wearable device may identify a tapping pattern (or a motion pattern) of the wearable device using one or more sensors (e.g., the acceleration sensor) included in the wearable device. For example, the tapping pattern of the wearable device may include a pattern (or movement) that occurs by repeating an impact greater than or equal to designated magnitude in the wearable device in a designated time interval. The wearable device may identify a function corresponding to the tapping pattern of the wearable device. The wearable device may perform the identified function or transmit a signal related to the identified function to an external electronic device connected with the wearable device to perform the identified function.

An operation of the wearable device according to the above-described embodiment may be described below. The wearable device described below may correspond to the electronic device 101 of FIG. 1. In the following specification, for convenience of a description, a structure and the operation of the wearable device, which is formed in a ring shape will be described. However, it is not limited thereto. For example, the wearable device may be implemented in various forms that are wearable by the user, such as a smart watch, a smart band, a smart ring, a wireless earphone, or smart glasses.

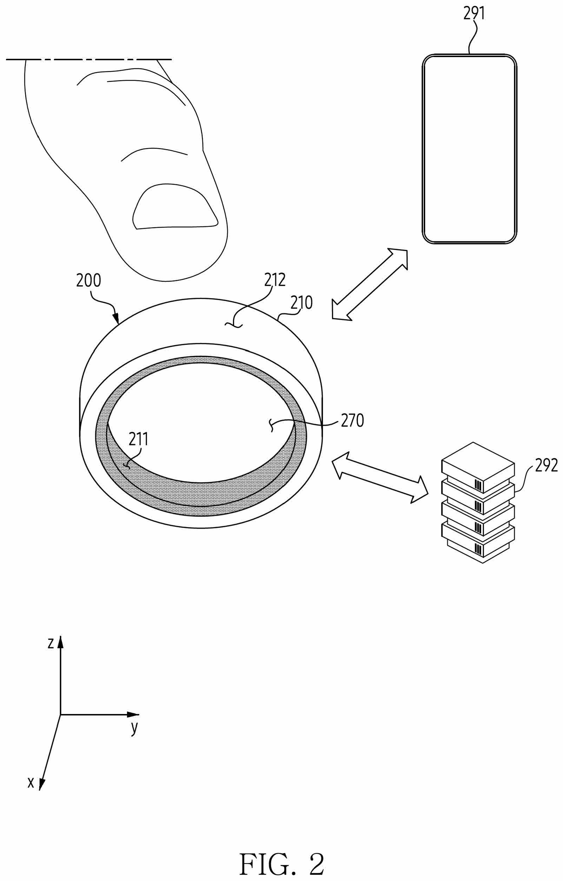

FIG. 2 illustrates an example of a wearable device and external electronic devices connected with the wearable device, according to an embodiment.

Referring to FIG. 2, a wearable device 200 may include a housing 210 including a first surface 211 facing a part (e.g., a finger) of a body of a user and a second surface 212 opposite to the first surface 211. For example, the wearable device 200 may include a housing 210 with ring-shaped. As an example, the wearable device 200 may be configured in a ring shape.

According to an embodiment, the wearable device 200 may be referred to as the wearable device that may be worn by the user. The wearable device 200 may be worn on the part (e.g., the finger) of the body of the user. For example, the wearable device 200 may be worn on the part of the body of the user. For example, the wearable device 200 may be fastened to the part of the body of the user. For example, the wearable device 200 may be detachable from the part of the body of the user. For example, the wearable device 200 may have a shape corresponding to the part of the body of the user to be worn on the part of the body of the user.

For example, the wearable device 200 may be contacted with the part of the body of the user by being worn by the user. For example, the wearable device 200 may be configured to obtain information on the user through the part of the body of the user by being worn by the user. For example, the wearable device 200 may provide the information on the user through the wearable device 200 and/or external electronic devices 291 and 292 connected with the wearable device 200. However, it is not limited thereto.

According to an embodiment, at least a portion of the first surface 211 may be contacted with the part of the body of the user in a case that the wearable device 200 is worn by the user. For example, the first surface 211 may surround the part of the body of the user by which the wearable device 200 is worn. For example, the first surface 211 may cover the part of the body of the user by which the wearable device 200 is worn. For example, when the wearable device 200 is worn by the user, the first surface 211 may be configured such that the wearable device 200 is fastened to the part of the body by pressurizing the part of the body of the user. For example, the first surface 211 may be deformable by the part of the body of the user. For example, the wearable device 200 may provide the information on the user through the first surface 211 based on a haptic technology.

For example, the second surface 212 may form an exterior of the wearable device 200 together with the first surface 211. For example, the second surface 212 may form a ring-shaped housing 210 together with the first surface 211. For example, the second surface 212 may be a surface spaced apart from the part of the body of the user in a case that the wearable device 200 is worn by the user. For example, the first surface 211 may be referred to as an inner circumference surface of the housing 210. The second surface 212 opposite to the first surface 211 may be referred to as an outer circumference surface of the housing 210.

For example, the second surface 212 may be exposed to an outside in a state that the wearable device 200 is worn by the user. The second surface 212 may be composed of at least one of titanium, stainless steel, and ceramic. The second surface 212 may be composed of a material for protecting against an external impact and/or a scratch. According to an embodiment, the second surface 212 may be coated with an additional material to protect a color of the wearable device 200 and/or the exterior of the wearable device 200.

For example, the first surface 211 may be composed of the same and/or a similar material as the second surface 212. According to an embodiment, the at least a portion of the first surface 211 may be composed of at least one of a molding material, a transparent plastic, and/or glass for obtaining data. According to an embodiment, the at least a portion of the first surface 211 may be composed of a metal for identifying a biological signal.

According to an embodiment, the wearable device 200 may further include a hole 270 formed by the first surface 211 to pass through the part of the body of the user when the wearable device 200 is worn by the user. For example, in a case that the wearable device 200 is worn by the user, the hole 270 may be penetrated by the part of the body of the user. The wearable device 200 may be configured to be fastened to the part of the body of the user in a case that the user wears the wearable device 200 by including the hole 270 configured to pass through the part of the body of the user.

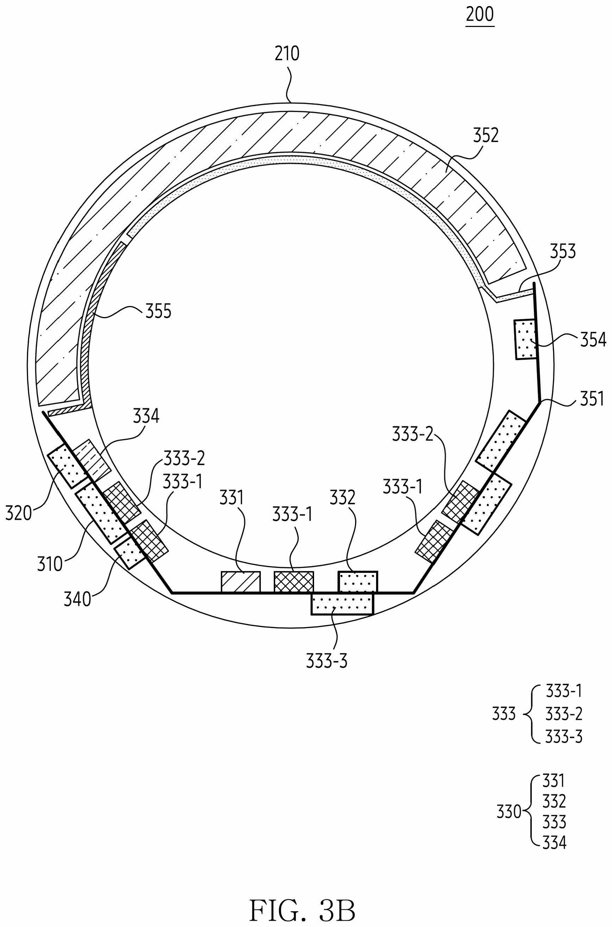

According to an embodiment, the wearable device 200 may further include one or more components between the first surface 211 and the second surface 212. For example, the wearable device 200 may include communication circuitry, one or more sensors, and/or a processor between the first surface 211 and the second surface 212. The disposition of one or more components will be described later in FIG. 3B.

According to an embodiment, the wearable device 200 may be connected with the external electronic device 291 and/or the external electronic device 292. For example, the user of the external electronic device 291 may be the same as the user of the wearable device 200. As an example, the external electronic device 291 may be a device connected with the wearable device 200 at a short distance and used by the same user. For example, the user of the external electronic device 292 may not be the same as the user of the wearable device 200. As an example, the external electronic device 292 may be a server configured for an emergency SOS service. However, it is not limited thereto.

For example, the wearable device 200 may be connected with the external electronic device 291. As an example, the wearable device 200 may be connected with the external electronic device 291 using various radio access technologies (RATs) (e.g., Bluetooth communication and a wireless LAN (WLAN)). For example, the wearable device 200 may control the external electronic device 291 or may be controlled by the external electronic device 291. As an example, the wearable device 200 may receive a request for the information on the user from the external electronic device 291. The wearable device 200 may transmit the information on the user to the external electronic device 291 based on the request received from the external electronic device 291.

For example, the wearable device 200 may be connected with the external electronic device 292. As an example, the wearable device 200 may be connected with the external electronic device 292 using various radio access technologies (RATs) (e.g., satellite communication and cellular communication). As an example, the wearable device 200 may be directly connected with the external electronic device 292. For example, the wearable device 200 may transmit the information on the user to the external electronic device 292. The external electronic device 292 may store the information on the user or provide a service (e.g., the emergency SOS service) for the user. According to an embodiment, the wearable device 200 may also be connected with the external electronic device 292 through the external electronic device 291.

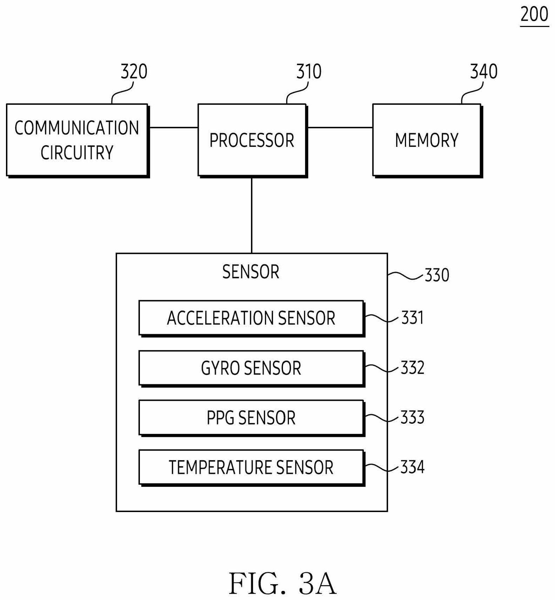

FIG. 3A illustrates an example of a simplified block diagram of a wearable device according to an embodiment.

Referring to FIG. 3A, a wearable device 200 may correspond to the electronic device 101 of FIG. 1.

According to an embodiment, the wearable device 200 may include a processor 310, communication circuitry 320, a sensor 330, and/or memory 340. According to an embodiment, the wearable device 200 may include at least one of the processor 310, the communication circuitry 320, the sensor 330, and/or the memory 340. For example, at least some of the processor 310, the communication circuitry 320, the sensor 330, and/or the memory 340 may be omitted according to an embodiment.

According to an embodiment, the processor 310 may correspond to the processor 120 of FIG. 1. The processor 310 may be operatively (or operably) coupled with or connected with the communication circuitry 320, the sensor 330, and the memory 340. The processor 310 being operatively (or operably) coupled with or connected with the communication circuitry 320, the sensor 330, and the memory 340 may mean that the processor 310 may control the communication circuitry 320, the sensor 330, and the memory 340. For example, the communication circuitry 320, the sensor 330, and the memory 340 may be controlled by the processor 310.

Although illustrated based on different blocks, an embodiment is not limited thereto, and a portion (e.g., at least a portion of the processor 310, the communication circuitry 320, and the memory 340) of hardware of FIG. 3A may be included in a single integrated circuit such as a system on a chip (SoC).

According to an embodiment, the processor 310 may be configured with at least one processor. For example, the processor 310 may be configured with a main processor that performs high-performance processing and an auxiliary processor that performs low-power processing. At least a portion of the sensor 330 may be connected to the auxiliary processor. The at least a portion of the sensor connected to the auxiliary processor may obtain data related to a user for 24 hours. According to an embodiment, one of the main processor and the auxiliary processor may be activated according to a state and/or an operation of the wearable device 200. As an example, in a state that a battery of the wearable device 200 is insufficient, the auxiliary processor may be activated. As an example, in a state that accurate data related to the user is required, the main processor may be activated.

According to an embodiment, the processor 310 may include a hardware component for processing data based on one or more instructions. The hardware component for processing the data may include, for example, an arithmetic and logic unit (ALU), a field programmable gate array (FPGA), and/or a central processing unit (CPU).

For example, the processor 310 may include an application processor, a supplementary processor (e.g., a sensor hub, a microcontroller unit (MCU), a central processor unit (CPU), a neural processing unit (NPU), and a graphic processing unit (GPU)), and/or a processor for an IoT (e.g., a processor integrated with a communication module).

According to an embodiment, the processor 310 may determine a timing of an operation of the sensor 330. The processor 310 may control the operation of the sensor 330. The processor 310 may process information obtained from the sensor 330. For example, the processor 310 may identify a tapping pattern of the wearable device 200 using the sensor 330. The processor 310 may identify the tapping pattern of the wearable device 200 based on data obtained using the sensor 330. An embodiment for identifying the tapping pattern of the wearable device 200 based on the data obtained using the sensor 330 will be described below.

According to an embodiment, the wearable device 200 may include the communication circuitry 320. The communication circuitry 320 may correspond to at least a portion of the communication module 190 of FIG. 1. For example, the communication circuitry 320 may be used for various radio access technologies (RATs). For example, the communication circuitry 320 may be used to perform Bluetooth communication, a wireless local area network (WLAN) communication, Zigbee communication, near field communication (NFC), ultra wide band (UWB) communication, or ANT+ communication. For example, the communication circuitry 320 may be used to perform cellular communication. For example, the processor 310 may establish a connection with an external electronic device (e.g., the external electronic device 291 and the external electronic device 292 of FIG. 2) through the communication circuitry 320. For example, the processor 310 may identify (or measure) a position of the wearable device 200 based on a wireless signal (e.g., a global positioning system (GPS) signal) received from or transmitted to, using the communication circuitry 320. According to an embodiment, the communication circuitry 320 may be configured to be integrated with the processor 310.

For example, the processor 310 may transmit data to the external electronic device (e.g., the external electronic device 291) connected with the wearable device 200 using the communication circuitry 320. The processor 310 may transmit information on the tapping pattern to the external electronic device based on a request of information on the tapping pattern (or data on the tapping) from the external electronic device using the communication circuitry 320. According to an embodiment, in a case that the user is in an emergency situation, the processor 310 may transmit (e.g., broadcast) a signal (e.g., a Bluetooth low energy (BLE) signal) for a SOS to a peripheral electronic device using the communication circuitry 320.

According to an embodiment, the wearable device 200 may include the sensor 330. The sensor 330 may be used to obtain various information. For example, the sensor 330 may be used to obtain data related to a body of the user and/or data related to the tapping. As an example, the sensor 330 may be used to obtain the user's body temperature data (or body temperature information), heart rate data (or heart rate information), and/or motion data (or motion information). For example, the sensor 330 may be configured with at least one sensor. The sensor 330 may include the at least one sensor. For example, the sensor 330 may correspond to a sensor module 176 of FIG. 1.

For example, the sensor 330 may include an acceleration sensor 331. The acceleration sensor 331 may be used to identify a change in acceleration of the wearable device 200. As an example, the acceleration sensor 331 may identify (or measure or detect) the acceleration of the wearable device 200 in three directions of an x-axis, a y-axis, and a z-axis. As an example, the acceleration sensor 331 may be set to identify acceleration approximately four times or more of acceleration of gravity on any axis. As an example, the acceleration sensor 331 may have a resolution of designated magnitude (e.g., 16 bits).

For example, the sensor 330 may include a gyro sensor 332. The gyro sensor 332 may identify (or measure or detect) angular velocity of the wearable device 200 in the three directions of the x-axis, the y-axis, and the z-axis. According to an embodiment, the wearable device 200 may include an inertial sensor including the acceleration sensor 331 and the gyro sensor 332.

For example, the wearable device 200 may identify at least one of the data related to the tapping, data related to a gesture of the user, data related to an amount of impact, data related to an orientation of the wearable device 200, and/or activity information (e.g., sitting, moving, or sports activity) of the user by using at least one of the acceleration sensor 331 and/or the gyro sensor 332.

For example, the sensor 330 may include a photoplethysmography (PPG) sensor 333. The PPG sensor 333 may be used to measure a pulse (or a change in an amount of blood in a blood vessel) by identifying an amount of change in an amount of photosensitive of light according to a change in a blood vessel volume. The PPG sensor 333 may include one or more photodiodes (PDs) and one or more light emitting diodes (LEDs). For example, the PPG sensor 333 may be used to identify a change in a blood flow rate in the blood vessel during a heartbeat. The PPG sensor 333 may identify the change in the blood flow rate in the blood vessel during the heartbeat in a state that an optical sensor is contacted to a skin over a peripheral blood vessel. The processor 310 may identify the blood flow rate and identify an amount of change in the blood flow rate based on a PPG signal and a waveform.

For example, the PPG sensor 331 may include a transmissive PPG sensor and/or a reflective PPG sensor.

As an example, the PPG sensor 331 may output light toward the skin of the user through one of the LED (e.g., green, red, or infrared (IR)), laser, and a vertical cavity surface emitting laser (VCSEL). The PPG sensor 331 may identify light reflected by and/or transmitted from the skin of the user through at least one of the PD and/or a complementary metal oxide semiconductor (CMOS) camera. The PPG sensor 331 may store a value identified through an analog to digital converter (ADC) in the memory 340 (or a buffer) based on the reflected and/or transmitted light.

As an example, the transmissive PPG sensor may identify light that has passed through the blood vessel through the PD disposed on an opposite side of the LED. The transmissive PPG sensor may identify the blood flow rate of the user based on an intensity of the light that has passed through the blood vessel. As an example, the reflective PPG sensor may output light toward the skin of the user through the LED. The reflective PPG sensor may identify at least some received light reflected by the blood vessel through the PD disposed on substantially the same surface as the LED. The reflective PPG sensor may identify the blood flow rate of the user based on an intensity of the light reflected by the blood vessel. For example, a multi-light source may be used as the LED. For example, green light, which is a complementary color to blood, may be used as the LED.

For example, the sensor 330 may include a temperature sensor 334. The temperature sensor 334 may be used to identify (or measure) a skin temperature on a part (e.g., a wrist or a forehead) of the body of the user. For example, the temperature sensor 334 may include a contact type temperature sensor and a non-contact type temperature sensor.

Although not illustrated, the sensor 330 may further include various sensors for obtaining (or identifying, measuring, or detecting) various data related to the user and/or the wearable device 200.

According to an embodiment, the wearable device 200 may include the memory 340. The memory 340 may be used to store information or data. For example, the memory 340 may be used to store data obtained from the user. For example, the memory 340 may correspond to the memory 130 of FIG. 1. For example, the memory 340 may be a volatile memory unit or volatile memory units. For example, the memory 340 may be a non-volatile memory unit or non-volatile memory units. As another example, the memory 340 may be another type of computer readable medium, such as a magnetic disk or an optical disk. For example, the memory 340 may store data obtained based on an operation (e.g., an algorithm performing operation) performed by the processor 310. For example, the memory 340 may store data (e.g., the data related to the tapping) obtained by the sensor 330. According to an embodiment, the memory 340 may be configured in a form integrated with the processor 310.

For example, the processor 310 may store information on the tapping pattern identified based on data obtained from the sensor 330 (e.g., the acceleration sensor 331) in the memory 340. As an example, the processor 310 may store information on the tapping pattern based on a designated time interval. For example, the processor 310 may store one or more custom tapping patterns set by the user and/or one or more preset tapping patterns basically stored in the wearable device 200 in the memory 340. For example, the processor 310 may store one tapping pattern in association with one function. As an example, the processor 310 may store the function corresponding to the tapping pattern in the memory 340 in a form of a table.

Although not illustrated in FIG. 3A, the wearable device 200 may further include various components. For example, the wearable device 200 may further include at least one of an antenna, a power management integrated circuit (PMIC), the battery, and a flexible printed circuit board (FPCB).

FIG. 3B is an example of a partial cross-sectional view of a wearable device according to an embodiment.

Referring to FIG. 3B, a wearable device 200 (e.g., the electronic device 101 of FIG. 1) may be formed in a ring shape. For example, a housing 210 of the wearable device 200 may be formed in a ring form that may be worn on a finger of a user. In FIGS. 2 and 3B, the ring-shape wearable device 200 having a smooth surface is illustrated as an example, but is not limited thereto. For example, the wearable device 200 may be implemented as a housing including a plurality of planes. For example, the ring-shape wearable device 200 having a non-smooth surface may also be understood as an embodiment of the present disclosure.

According to an embodiment, the ring-shape housing 210 may include a first surface (e.g., the first surface 211 of FIG. 2) contacted with a body of the user in a state of being worn by the user, a second surface (e.g., the second surface 212 of FIG. 2) exposed to an outside, and a lateral surface between the first surface 211 and the second surface 212. For example, between the first surface 211 and the second surface 212, a space for including (or disposing) at least one of components (e.g., a processor 310, communication circuitry 320, a sensor 330, and memory 340) may be included.

According to an embodiment, a PCB 351 may be disposed between the first surface 211 and the second surface 212 of the wearable device 200. For example, the processor 310, the communication circuitry 320, an acceleration sensor 331, a gyro sensor 332, a PPG sensor 333, a temperature sensor 334, the memory 340, and/or a PMIC 354 may be disposed on the PCB 351. For example, the PCB 351 may be composed of a rigid region and a flexible region. As an example, the rigid region may be referred to as a rigid flexible printed circuit board (RFPCB). As an example, the flexible region may be referred to as a flexible printed circuit board (FPCB).

For example, the PPG sensor 333 may include one or more light emitting circuitry 333-1, one or more light receiving circuitry 333-2, and control circuitry 333-3. For example, the one or more light emitting circuitry 333-1 and the one or more light receiving circuitry 333-2 may be disposed toward the first surface 211. As an example, the control circuitry 333-3 may be disposed toward the second surface 212.

For example, the PMIC 354 may be used to manage power of the wearable device 200. The PMIC 354 may be used in the wearable device 200 to provide (or distribute) the power to components requiring the power. Through a charging interface 353, the PMIC 354 may support a wired charging method (e.g., a terminal and a pogo pin) or a wireless charging method (e.g., a wireless power consortium (WPC) and an NFC) for charging the wearable device 200.

According to an embodiment, a battery 352 may be disposed between the first surface 211 and the second surface 212 of the wearable device 200. The battery 352 may be configured with at least one battery (or a battery pack). For example, the battery 352 may be configured such that the at least one battery is connected in series and/or in parallel. For example, the battery 352 may be configured with a flexible battery pack. For example, the battery 352, which is a secondary battery, may be charged and/or discharged. For example, materials composing the battery 352 may be variously configured with. As an example, the material composing the battery 352 may include at least one of a lithium ion and mercury.

According to an embodiment, an antenna 355 may be disposed between the first surface 211 and the second surface 212 of the wearable device 200. For example, the antenna 355 may be configured with a single antenna and/or a plurality of segment antennas. According to an embodiment, the antenna 355 may be configured as a portion of the housing 210 of the wearable device 200. For example, the antenna 355 may be electrically connected with the communication circuitry 320 through the PCB 351.

Although not illustrated, the wearable device 200 may further include various components in addition to the illustrated components. For example, the wearable device 200 may include a display. The display may be disposed on an outer surface of the housing 210.

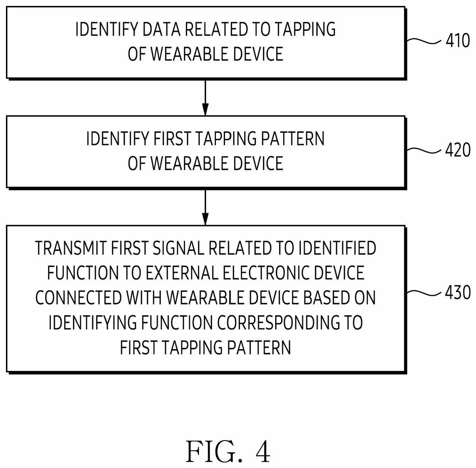

FIG. 4 illustrates a flowchart of an operation of a wearable device according to an embodiment. A wearable device 200 may perform operations illustrated in FIG. 4. Hereinafter, the operations illustrated in FIG. 4 will be described as being performed by a processor (e.g., the processor 310 of FIG. 3A). In the following embodiment, each of the operations may be sequentially performed, but is not necessarily performed sequentially. For example, an order of each of the operations may be changed, and at least two operations may be performed in parallel.

Referring to FIG. 4, in operation 410, a processor 310 may identify data related to tapping. For example, the wearable device 200 may identify the data related to the tapping using a sensor 330 during a designated time interval.

According to an embodiment, the processor 310 may identify the data related to the tapping during the designated time interval. For example, the designated time interval may be one of a plurality of time intervals set based on a sliding window scheme. The processor 310 may identify the plurality of time intervals based on the sliding window scheme. For example, at least some of the plurality of time intervals may overlap each other. For example, a first time interval may be set for 10 milli-seconds [ms] from a designated timing. A second time interval may be set for 10 [ms] from a timing at which 1 [ms] has elapsed from the designated timing.

According to an embodiment, the processor 310 may receive a request for identifying a tapping pattern of the wearable device 200 from an external electronic device. In response to the request for identifying the tapping pattern, the processor 310 may identify the data related to the tapping by using the sensor 330 during the designated time interval from a timing at which the request is received to identify the tapping pattern of the wearable device 200.

According to an embodiment, the data related to the tapping may include data related to an orientation of the wearable device 200 and data related to a change in acceleration of the wearable device 200. For example, the processor 310 may identify the data related to the orientation of the wearable device 200 and the data related to the change in the acceleration of the wearable device 200 by using at least one of an acceleration sensor 331 and/or a gyro sensor 332.

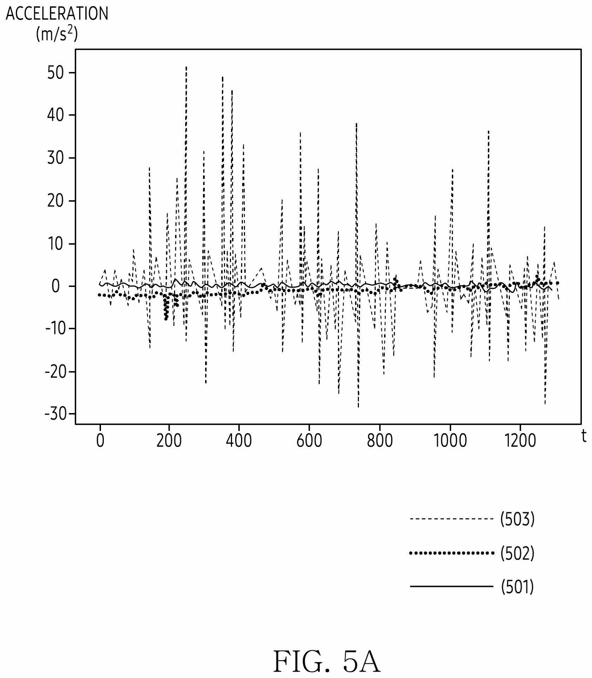

For example, a specific example in which the processor 310 identifies the data related to the tapping by using the acceleration sensor 331 will be described later in FIGS. 5A to 5D.

According to an embodiment, the processor 310 may identify information on a body of a user using the sensor 330. The processor 310 may identify that a state of the user is in a designated state based on the information on the body of the user. The processor 310 may identify the data related to the tapping while the state of the user is in the designated state.

For example, the processor 310 may identify that a body temperature of the user is greater than or equal to a designated body temperature (e.g., approximately 39 degrees). The processor 310 may identify that the state of the user is in a dangerous state. The processor 310 may identify the data related to the tapping to perform an emergency SOS, based on identifying that the state of the user is in the dangerous state.

For example, the processor 310 may identify that the user is boarding a vehicle using the sensor 330. Based on identifying that the user is boarding the vehicle, the processor 310 may identify the data related to the tapping to perform one of a plurality of functions that may be performed in the vehicle.

In operation 420, the processor 310 may identify a first tapping pattern of the wearable device 200. For example, the processor 310 may identify the first tapping pattern of the wearable device 200 based on the identified data.

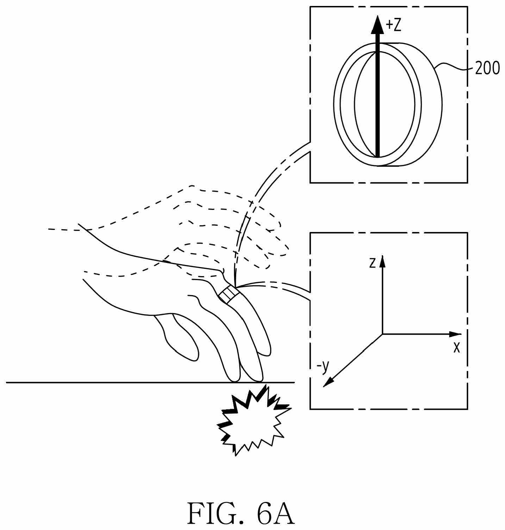

According to an embodiment, the processor 310 may identify the first tapping pattern among a plurality of tapping patterns based on the identified data. The plurality of tapping patterns may include a tapping pattern tapping an upper surface of an external object using the part of the body of the user in a state that the user wears the wearable device 200. The plurality of tapping patterns may include a tapping pattern tapping a side surface of the external object using the part of the body of the user in the state that the user wears the wearable device 200. As an example, the tapping pattern tapping the upper surface of the external object using the part of the body of the user may be referred to as knocking. As an example, the tapping pattern tapping the side surface of the external object using the part of the body of the user may be referred to as bumping. A specific example of the plurality of tapping patterns will be described later in FIGS. 6A to 6D. According to an embodiment, the processor 310 may identify whether the user of the wearable device 200 taps the upper surface of the external object or the side surface of the external object, using the part of the body, based on the orientation of the wearable device 200.

According to an embodiment, the processor 310 may store information on the first tapping pattern in memory (e.g., the memory 340 of FIG. 3A). For example, the processor 310 may store the information on the first tapping pattern in the memory 340 based on the sliding window scheme. The processor 310 may store information on an amount of impact (or an intensity) and information on the identified timing in the memory 340.

In operation 430, the processor 310 may transmit a first signal related to the identified function to the external electronic device connected with the wearable device 200 based on identifying a function corresponding to the first tapping pattern.

According to an embodiment, the processor 310 may identify the function corresponding to the first tapping pattern. For example, each of the plurality of tapping patterns may correspond to different functions. For example, one of the plurality of tapping patterns may correspond to a control (e.g., turning on and turning off) of lighting connected with the wearable device 200. One of the plurality of tapping patterns may correspond to execution of an application of the external electronic device (e.g., the external electronic device 291 of FIG. 2).

According to an embodiment, the plurality of tapping patterns may be identified based on the information on the body of the user and information on an environment of the wearable device 200. For example, each of the plurality of tapping patterns may correspond to the different functions according to a situation. As an example, in a state that the body temperature of the user is higher than a designated temperature, each of the plurality of tapping patterns may correspond to a function for the emergency SOS. As an example, in a state that the user is driving, each of the plurality of tapping patterns may correspond to a function for controlling the vehicle.

For example, the processor 310 may identify the plurality of tapping patterns based on the information on the body of the user and the information on the environment of the wearable device 200. The processor 310 may identify the first tapping pattern among the plurality of tapping patterns. The processor 310 may identify the function corresponding to the first tapping pattern.

According to an embodiment, the processor 310 may transmit the first signal related to the identified function to the external electronic device (e.g., the external electronic device 291 or the external electronic device 292) connected with the wearable device 200.

For example, the first signal may be set to cause the external electronic device to perform the function identified based on the first tapping pattern. The processor 310 may transmit the first signal causing the external electronic device to perform the function identified based on the first tapping pattern, to the external electronic device. As an example, the function identified based on the first tapping pattern may be to perform an outgoing call to an emergency contact. The processor 310 may transmit the first signal to cause the external electronic device to transmit a second signal for an outgoing call to a device corresponding to the emergency contact.

For example, the first signal may be transmitted to perform the function identified based on the first tapping pattern. The processor 310 may transmit the first signal to the external electronic device to perform the identified function. As an example, the function identified based on the first tapping pattern may be a function for notifying an emergency state of the user. The processor 310 may transmit the first signal to the external electronic device (e.g., a server) configured for an emergency SOS service to indicate that the user is in the emergency state.

According to an embodiment, before identifying the first tapping pattern, the processor 310 may identify a second tapping pattern and a function corresponding to the second tapping pattern. The processor 310 may store the function corresponding to the second tapping pattern in association with the second tapping pattern. Based on identifying that the first tapping pattern corresponds to the second tapping pattern, the processor 310 may identify the function corresponding to the second tapping pattern as the function corresponding to the first tapping pattern.

FIGS. 5A to 5D illustrate an example of data identified through an acceleration sensor according to an embodiment.

Referring to FIG. 5A, a processor (e.g., the processor 310 of FIG. 3A) may identify a change in acceleration of a wearable device 200 through an acceleration sensor (e.g., the acceleration sensor 331 of FIG. 3A). For example, the processor 310 may identify the change in acceleration through the acceleration sensor 331 based on a designated sampling rate (e.g., 100 Hz).

According to an embodiment, the processor 310 may sample an acceleration value based on the sampling rate (e.g., 100 Hz) designated through the acceleration sensor 331. A graph 501, a graph 502, and a graph 503 indicate a change in an acceleration value according to a time obtained through the acceleration sensor 331. For example, the graph 501 indicates a change in an acceleration value according to a time with respect to an x-axis direction. The graph 502 indicates a change in an acceleration value according to a time with respect to a y-axis direction. The graph 503 indicates a change in an acceleration value according to a time with respect to a z-axis direction. For example, each of the x-axis direction, the y-axis direction, and the z-axis direction may correspond to the x-axis direction, the y-axis direction, and the z-axis direction of FIG. 2.

The processor 310 may set an east north up (ENU) coordinate system with respect to a gravity direction (e.g., the -z-axis direction of FIG. 2). The processor 310 may identify magnitude of the acceleration based on the ENU coordinate system.

For example, the processor 310 may identify the magnitude of the acceleration using acceleration values identified on each of an x-axis, a y-axis, and a z-axis. For example, the magnitude of the acceleration may be set as illustrated in Equation 1 below. [Equation 1] $M = \sqrt{{x}^{2} + {y}^{2} + {z}^{2}} .$

Equation 1 above is merely an example for helping understanding, and is not limited thereto, and may be modified, applied, or extended in various methods.

Referring to Equation 1, the M is the magnitude of the acceleration. The x is the acceleration value in the x-axis direction. The y is the acceleration value in the y-axis direction. The z is the acceleration value in the z-axis direction. For example, a unit of the above-described acceleration value (or the magnitude of the acceleration) may be [m/s²].

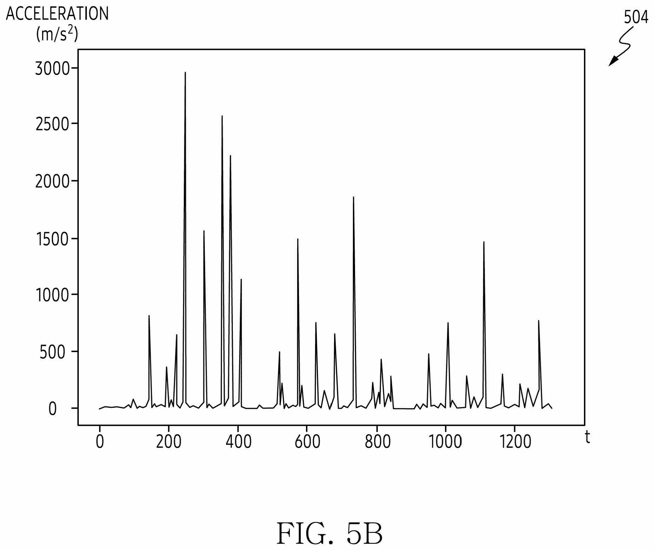

Referring to FIG. 5B, the processor 310 may identify a graph 504 indicating a change in magnitude M of acceleration according to a time based on Equation 1 described above.

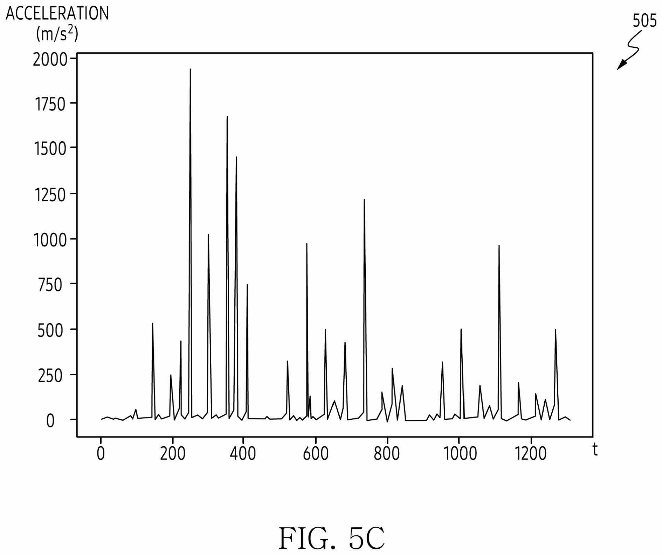

Referring to FIG. 5C, the processor 310 may remove noise using a low pass filter (LPF). The processor 310 may identify a graph 505 indicating a change in magnitude of acceleration according to a time, from which the noise has been removed, using the LPF.

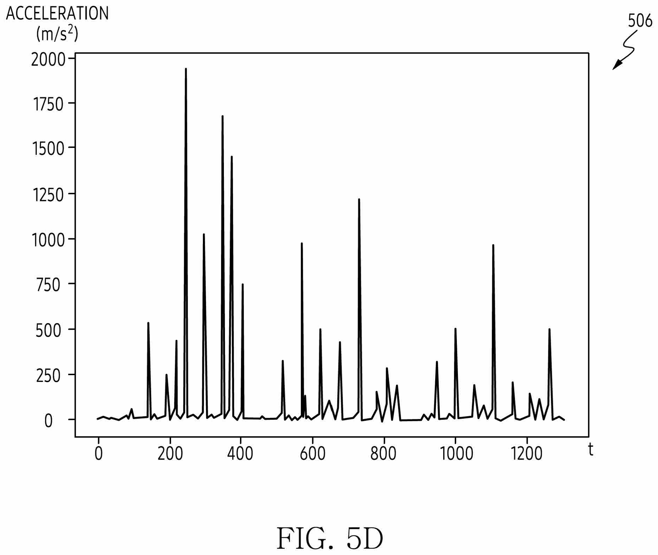

Referring to FIG. 5D, the processor 310 may identify values having magnitude of acceleration greater than or equal to a threshold value. For example, the processor 310 may identify peak values for the graph 505. The processor 310 may identify a graph 506 configured with the peak values for the graph 505. The processor 310 may identify, based on the graph 506, the sampled time and magnitude of acceleration as illustrated in Table 1.

**[Table 1]**

| | | | | |
|---|---|---|---|---|
| **sample #** | **144** | **195** | **224** | **...** |
| **magnitude** | **535.1208** | **247.5662** | **433.0177** | **...** |

Referring to Table 1, the processor 310 may identify a time at which a peak occurs and magnitude of acceleration of the corresponding time. The processor 310 may store the time at which the peak occurs and the magnitude of the acceleration of the corresponding time in memory (e.g., the memory 340 of FIG. 3A) as illustrated in Table 1.

The processor 310 may identify at least one timing at which the peak occurs based on the change in the magnitude of the acceleration according to the time. The processor 310 may identify that a user has tapped an external object through a part of a body of the user at the at least one timing.

For example, in a state that the wearable device 200 is worn by the user, there is a high probability that a movement displacement will be large and a high-frequency motion will be detected. Accordingly, according to the above-described embodiment, an operation not associated with a tapping operation may be filtered.

According to the above-described embodiment, the processor 310 may identify (e.g., monitor) an amount of impact identified in the wearable device 200. The processor 310 may identify a time interval between peak-to-peak of the amount of impact. The processor 310 may identify a tapping pattern (or an impact pattern configuring the tapping pattern) based on the number of tapping in a designated time, the amount of impact, and/or the time interval between the peak-to-peak of the amount of impact.

According to an embodiment, the processor 310 may identify an orientation of the wearable device 200 using the acceleration sensor 331. For example, based on the orientation of the wearable device 200, the processor 310 may identify whether the user taps an upper surface of the external object using the part (e.g., a finger) of the body. For example, based on the orientation of the wearable device 200, the processor 310 may identify whether the user taps a side surface of the external object by using the part of the body.

According to an embodiment, the processor 310 may identify that the user taps the external object with the part (e.g., the finger) of the body of the user based on the acceleration value in the z-axis direction among the acceleration values identified in each of the x-axis, the y-axis, and the z-axis. For example, in a case that the user taps the external object with the part (e.g., the finger) of the body of the user, the biggest change in acceleration in the z-axis direction may occur. Accordingly, the processor 310 may identify that the user taps the external object with the part (e.g., the finger) of the body of the user based on the acceleration value in the z-axis direction among the identified acceleration values.

According to an embodiment, the processor 310 may identify the orientation of the wearable device 200 using a gyro sensor (e.g., the gyro sensor 332 of FIG. 3A) (or a geomagnetic sensor). The processor 310 may identify information on a trajectory of movement of the wearable device 200 based on the orientation of the wearable device 200 identified using the gyro sensor 332 (or the geomagnetic sensor) and the change in the acceleration of the wearable device 200 identified using the acceleration sensor 331. The processor 310 may identify that the user taps the external object with the part (e.g., the finger) of the body of the user based on the information on the trajectory of the movement of the wearable device 200.

FIGS. 6A to 6D illustrate an example of a plurality of tapping patterns according to an embodiment.

Referring to FIGS. 6A to 6D, a processor (e.g., the processor 310 of FIG. 2A) may identify a tapping pattern based on an orientation and an impact pattern occurred in a designated time interval of a wearable device 200. For example, the tapping pattern may be configured based on the orientation and the impact pattern of the wearable device 200. For example, the tapping pattern (or the impact pattern) may be identified based on a tapping intensity and a tapping period.

For example, although in a case that impact patterns occurred in the designated time interval are substantially the same, a processor 310 may identify the impact patterns as different tapping patterns in a case that the orientation of the wearable device 200 is different. In a case that the orientation of the wearable device 200 is substantially the same and the impact pattern occurred in the designated time interval is different, the processor 310 may identify the impact patterns as different tapping patterns.

For example, the processor 310 may identify whether the impact pattern is the same and/or similar based on the number of tapping, an amount of impact, and/or a time interval between peak-to-peak of the amount of impact in a designated time.

For example, the processor 310 may identify the tapping pattern based on a position at which an impact acting on the wearable device 200 is applied and/or a direction in which the impact is applied to the wearable device 200 with respect to a gravity direction. In FIGS. 6A to 6D, an example in which the processor 310 identifies a motion based on the orientation of the wearable device 200 will be described.

Axes (e.g., an x-axis, a y-axis, and a z-axis) (e.g., the x-axis, the y-axis, and the z-axis of FIG. 2) of FIGS. 6A to 6D may indicate axes with respect to an external environment with respect to the orientation of the wearable device 200. The axes (e.g., the x-axis, the y-axis, and the z-axis of FIG. 2) identified in the wearable device 200 may be maintained, although in a case that the orientation of the wearable device 200 is changed.

Referring to FIG. 6A, a user of the wearable device 200 may tap an upper surface (e.g., a surface facing a direction opposite to a direction of gravity) of an external object by moving a part (e.g., a finger or a wrist) of a body of the user in a state that a back of the user's hand faces the direction opposite to the gravity direction. As the user taps the upper surface of the external object, an impact may be applied to the wearable device 200. For example, the impact may be applied to the wearable device 200 in a +z direction with respect to the wearable device 200. The processor 310 may identify that a state of the wearable device 200 identified based on the impact applied to the wearable device 200 in the +z direction and the orientation of the wearable device 200 is in a first state (e.g., a normal state). The processor 310 may identify a first tapping pattern among the plurality of tapping patterns based on identifying that the state of the wearable device 200 is in the first state (e.g., the normal state).

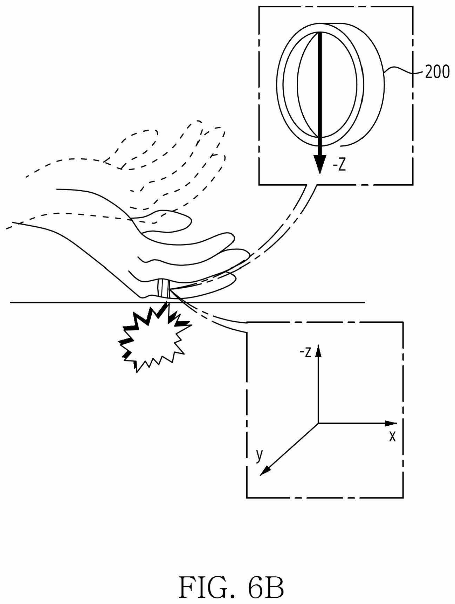

Referring to FIG. 6B, the user of wearable device 200 may tap the upper surface (e.g., the surface facing the direction opposite to the direction of gravity) of the external object by moving the part (e.g., the finger or the wrist) of the body of the user in the state that the back of the user's hand faces the gravity direction. As the user taps the upper surface of the external object, an impact may be applied to the wearable device 200. For example, the impact may be applied to the wearable device 200 in a -z direction with respect to the wearable device 200. The processor 310 may identify that a state of the wearable device 200 is in a second state (e.g., a flipped state) based on the impact applied to the wearable device 200 in the -z direction and the orientation of the wearable device 200. The processor 310 may identify a second tapping pattern among the plurality of tapping patterns based on identifying that the state of the wearable device 200 is in the second state (e.g., the flipped state).

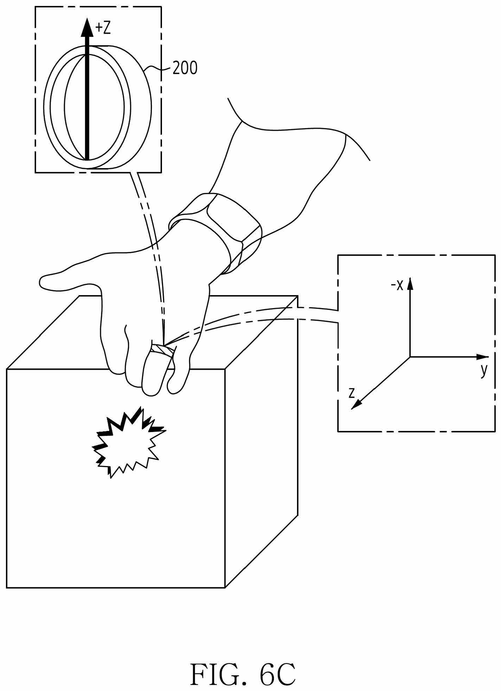

Referring to FIG. 6C, the user of the wearable device 200 may tap a side surface (e.g., a surface facing the +z direction) of the external object by moving the part (e.g., the finger) of the body of the user in a state that the back of the user's hand faces a direction (e.g., the +z direction among the axes identified in the wearable device 200) perpendicular to the gravity direction. As the user taps the side surface of the external object, an impact may be applied to the wearable device 200. For example, the impact may be applied to the wearable device 200 in the +z direction with respect to the wearable device 200. The processor 310 may identify that a state of the wearable device 200 is in a third state (e.g., a state rotated from the normal state) based on the impact applied to the wearable device 200 in the +z direction and the orientation of the wearable device 200. The processor 310 may identify a third tapping pattern among the plurality of tapping patterns based on identifying that the state of the wearable device 200 is in the third state (e.g., the state rotated from the normal state).

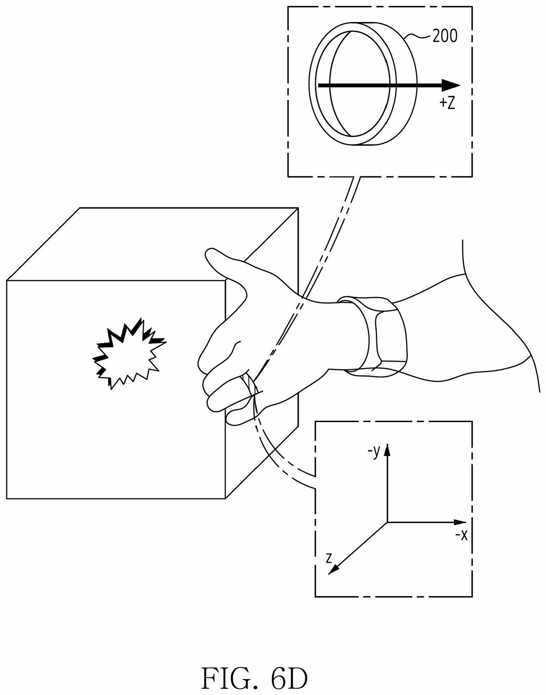

Referring to FIG. 6D, the user of the wearable device 200 may tap the side surface (e.g., a surface facing the direction perpendicular to the direction of gravity) of the external object by moving the part (e.g., the finger) of the body of the user in the state that the back of the user's hand faces the direction perpendicular to the gravity direction. As the user taps the side surface of the external object, an impact may be applied to the wearable device 200. For example, the impact may be applied to the wearable device 200 in the +z direction with respect to the wearable device 200. The processor 310 may identify that the impact occurs in the wearable device 200 in the +z direction, and that the state of the wearable device 200 identified based on the orientation of the wearable device 200 is in a fourth state (e.g., the state rotated from the normal state). The processor 310 may identify a fourth tapping pattern among the plurality of tapping patterns based on identifying that a state of the wearable device 200 is in the fourth state (e.g., the state rotated from the normal state) based on the impact occurred to the wearable device 200 in the +z direction and the orientation of the wearable device 200.

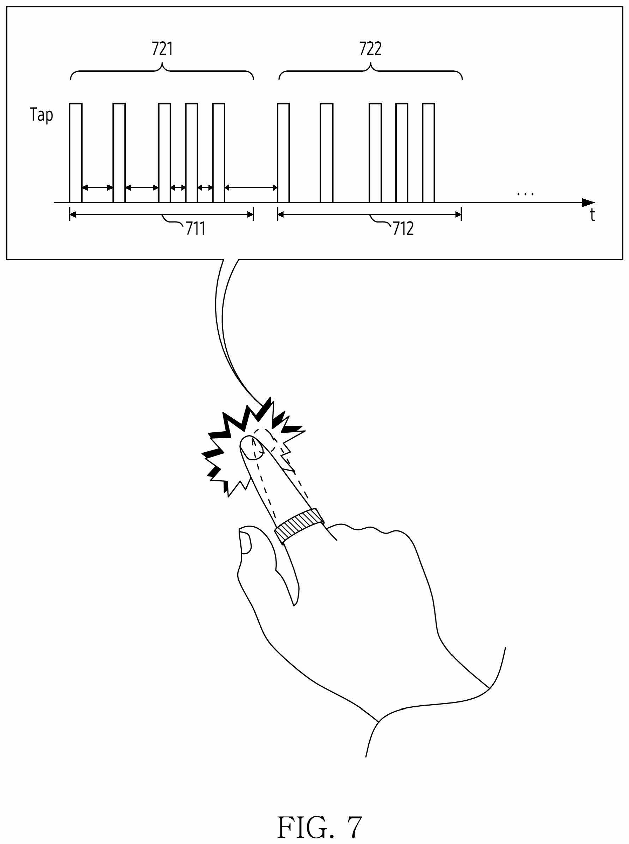

FIG. 7 illustrates an example of an operation of a wearable device for identifying an impact pattern according to an embodiment.

Referring to FIG. 7, a processor (e.g., the processor 310 of FIG. 3A) may identify a tapping pattern based on an orientation and an impact pattern occurred in a designated time interval of a wearable device 200. In FIG. 7, an example for a processor 310 to identify the tapping pattern based on the impact pattern occurred in the designated time interval will be described.

According to an embodiment, the processor 310 may identify an impact pattern 721 in a time interval 711. For example, the impact pattern 721 may be configured with one or more tapping inputs. For example, the processor 310 may identify the one or more tapping inputs of a designated pattern in the time interval 711. The processor 310 may identify the impact pattern 721 based on the one or more tapping inputs of the designated pattern.

According to an embodiment, the tapping pattern may be identified based on the impact pattern continuously repeated a designated number of times. For example, the processor 310 may identify the one or more tapping inputs in a time interval 712 after the one or more tapping inputs are identified in the time interval 711. The processor 310 may identify an impact pattern 722 in the time interval 712. The processor 310 may identify the impact pattern 721 in the time interval 711, and continuously, identify the impact pattern 722 in the time interval 712. The processor 310 may identify that the impact pattern 722 corresponds to the impact pattern 721. The processor 310 may continuously identify the impact patterns 721 and 722 having the same pattern. The processor 310 may identify the tapping pattern based on identifying that the impact pattern 722 corresponds to the impact pattern 721.

According to an embodiment, the processor 310 may identify repetitive impact patterns. As an example, the processor 310 may identify the time interval 711 in which the impact pattern 721 is identified based on identifying the impact pattern 721. After the impact pattern 721 is identified, the processor 310 may identify the impact pattern 722. The processor 310 may identify whether the impact pattern 722 corresponds to the impact pattern 721. For example, the processor 310 may identify a similarity between the impact pattern 721 and the impact pattern 722. The processor 310 may identify that substantially the same impact patterns 721 and 722 occur based on identifying that the similarity between the impact pattern 721 and the impact pattern 722 is greater than or equal to a designated value.

The above-described example describes an example in which the processor 310 identifies the tapping pattern in a case that substantially the same impact pattern is repeated twice, but is not limited thereto. According to an embodiment, in a case that substantially the same impact pattern is repeated five times, the processor 310 may identify the tapping pattern based on the impact pattern.

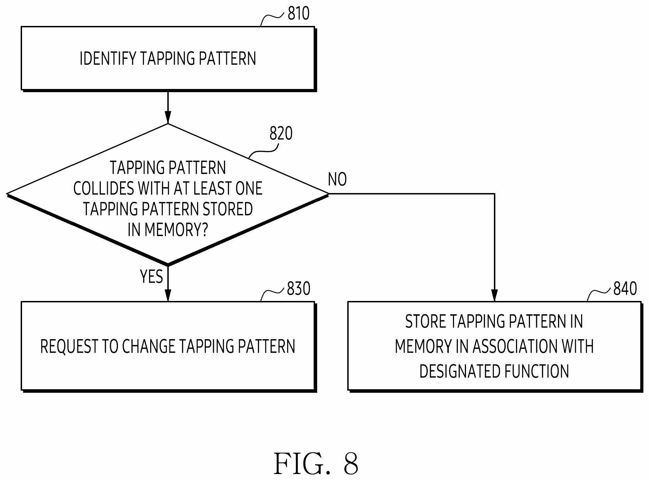

FIG. 8 illustrates a flowchart of an operation of a wearable device according to an embodiment. A wearable device 200 may perform operations illustrated in FIG. 8. Hereinafter, the operations illustrated in FIG. 8 will be described as being performed by a processor (e.g., the processor 310 of FIG. 3A). In the following embodiment, each of the operations may be sequentially performed, but is not necessarily performed sequentially. For example, an order of each of the operations may be changed, and at least two operations may be performed in parallel.

Referring to FIG. 8, operations 810 to 840 may be associated with an operation for setting a function corresponding to a tapping pattern in the wearable device 200.

In the operation 810, a processor 310 may identify the tapping pattern. For example, the processor 310 may identify the tapping pattern while the wearable device 200 operates in a mode for training the tapping pattern.

According to an embodiment, the processor 310 may request a user to set a tapping pattern corresponding to a designated function (or a first function). For example, the processor 310 may request the user to set a tapping pattern for execution (or performing) of the designated function (or the first function). As an example, the processor 310 may provide a notification for requesting to set the tapping pattern for the execution of the designated function. The processor 310 may identify data related to tapping based on the notification. The processor 310 may identify the tapping pattern based on the data related to the tapping.

According to an embodiment, the processor 310 may identify the tapping pattern using a model (e.g., a neural network model) indicated by a plurality of parameters. For example, the processor 310 may set the data related to the tapping as an input value of the model. The processor 310 may identify the tapping pattern based on an output value of the model.

For example, the processor 310 may identify the tapping pattern using the model. The model may be a model set to identify (or classify) the tapping pattern. The processor 310 may train the model to identify the tapping pattern. The model set to identify (or classify) the tapping pattern may have a low power consumption. As an example, the power consumption required for training and using the model may be less than or equal to a designated value.

In the operation 820, the processor 310 may identify whether the tapping pattern collides with at least one tapping pattern stored in memory (e.g., the memory 340 of FIG. 3A). For example, it may be a state that the at least one tapping pattern corresponding to at least one function may be stored in the memory 340 of the wearable device 200. The at least one tapping pattern may include one or more preset tapping patterns and/or one or more custom tapping patterns. As an example, the one or more preset tapping patterns may be a tapping pattern basically stored in the wearable device 200. The one or more preset tapping patterns may be a pre-stored tapping pattern when the wearable device 200 is shipped. As an example, the one or more custom tapping patterns may be a tapping pattern set by the user.

The processor 310 may identify whether the identified tapping pattern collides with the one or more custom tapping patterns. The processor 310 may identify whether the identified tapping pattern corresponds to a tapping pattern designated by the user. Based on identifying that the identified tapping pattern corresponds to one of the one or more custom tapping patterns, the processor 310 may identify that the identified tapping pattern is collided with the one or more custom tapping patterns. According to an embodiment, the number of one or more custom tapping patterns may be limited. For example, in order to decrease complexity of the model for identifying the tapping pattern, the number of one or more custom tapping patterns may be limited.

In a case that the identified tapping pattern is not collided with the one or more custom tapping patterns, the processor 310 may identify whether the identified tapping pattern is collided with the one or more preset tapping patterns. The processor 310 may identify whether the identified tapping pattern corresponds to one of the one or more preset tapping patterns basically stored in the wearable device 200. Based on identifying that the identified tapping pattern corresponds to one of the one or more preset tapping patterns basically stored in the wearable device 200, the processor 310 may identify that the identified tapping pattern is collided with the one or more preset tapping patterns.

In the above-described example, in a case that the identified tapping pattern is not collided with the one or more custom tapping patterns, the processor 310 has been described to perform an operation of identifying whether the identified tapping pattern is collided with the one or more preset tapping patterns, but is not limited thereto. For example, in a case that the identified tapping pattern is not collided with the one or more preset tapping patterns, the processor 310 may identify whether the identified tapping pattern is collided with the one or more custom tapping patterns.

In the operation 830, in a case that the identified tapping pattern collides with the at least one tapping pattern stored in the memory 340, the processor 310 may request to change the tapping pattern. Based on identifying that the identified tapping pattern corresponds to one of the one or more user tapping patterns and/or the one or more preset tapping patterns, the processor 310 may identify that the identified tapping pattern collides with the at least one tapping pattern stored in the memory 340. The processor 310 may request to change the tapping pattern based on identifying that the identified tapping pattern collides with the at least one tapping pattern stored in the memory 340.

For example, the processor 310 may request the user to re-enter the tapping pattern based on identifying that the identified tapping pattern is collided with the one or more custom tapping patterns.

For example, the processor 310 may request the user to re-enter the tapping pattern based on identifying that the identified tapping pattern is collided with the one or more preset tapping patterns.

In the operation 840, in a case that the identified tapping pattern does not collide with the at least one tapping pattern stored in the memory 340, the processor 310 may store the tapping pattern in the memory 340 in association with a designated function. For example, the processor 310 may execute (or perform) the designated function based on identifying that the tapping pattern identified based on the data related to the tapping corresponds to the tapping pattern stored in association with the designated function.

According to an embodiment, the wearable device 200 may not be able to provide a processing capacity for performing the above-described operations 810 to 840. The wearable device 200 may store the tapping pattern in association with the designated function by using an external electronic device connected with the wearable device 200.

For example, the external electronic device may request the user to set the tapping pattern for executing the designated function through the wearable device 200. The external electronic device may request the wearable device 200 to identify the data related to the tapping. The processor 310 may identify (or obtain) the data related to the tapping using a sensor (e.g., the sensor 330 of FIG. 3A). The processor 310 may transmit the data related to the tapping to the external electronic device. The external electronic device may set the data related to the tapping as the input value of the model for identifying the tapping pattern. The external electronic device may identify the tapping pattern by the output value of the model. The external electronic device may perform operations corresponding to the operations 810 to 840 based on identifying the tapping pattern.

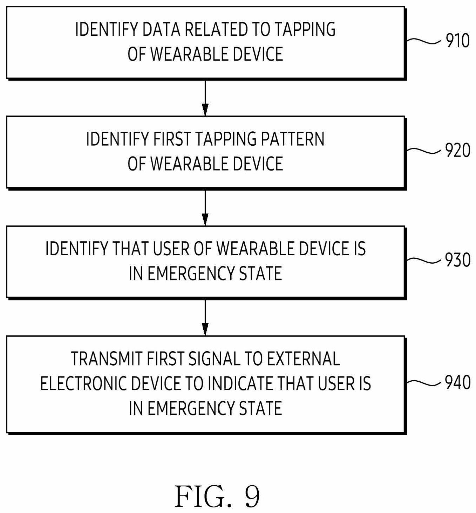

FIG. 9 illustrates a flowchart of an operation of a wearable device according to an embodiment. A wearable device 200 may perform operations illustrated in FIG. 9. Hereinafter, the operations illustrated in FIG. 9 will be described as being performed by a processor (e.g., the processor 310 of FIG. 3A). In the following embodiment, each of the operations may be sequentially performed, but is not necessarily performed sequentially. For example, an order of each of the operations may be changed, and at least two operations may be performed in parallel.

Referring to FIG. 9, in operation 910, a processor 310 may identify data related to tapping. For example, the wearable device 200 may identify the data related to the tapping using a sensor (e.g., the sensor 330 of FIG. 3A) during a designated time interval. The operation 910 may correspond to the operation 410 of FIG. 4.

In operation 920, the processor 310 may identify a first tapping pattern of the wearable device 200. For example, the processor 310 may identify the first tapping pattern of the wearable device 200 based on the identified data. The operation 920 may correspond to the operation 420 of FIG. 4.

In operation 930, the processor 310 may identify that a user of the wearable device 200 is in an emergency state. For example, the processor 310 may identify that the user of the wearable device 200 is in the emergency state based on identifying the first tapping pattern. For example, the processor 310 may identify that the user of the wearable device 200 is in the emergency state based on identifying the first tapping pattern continuously repeated a designated number of times. According to an embodiment, the processor 310 may identify whether the first tapping pattern is continuously identified based on identifying the first tapping pattern. The processor 310 may not perform the operation 930 in response to identifying the first tapping pattern. The processor 310 may perform the operation 930 based on the first tapping pattern being continuously identified.

According to an embodiment, the processor 310 may identify that the first tapping pattern is repeated. After identifying the first tapping pattern according to the operation 920, the processor 310 may repeatedly identify the first tapping pattern. The processor 310 may identify that the first tapping pattern is continuously repeated. The processor 310 may identify the first tapping pattern continuously repeated the designated number of times (e.g., three times).

For example, due to malfunction and/or a mistake of the user, the processor 310 may identify the first tapping pattern. In order to identify whether the user has intentionally performed a tapping operation corresponding to the first tapping pattern, the processor 310 may identify whether the first tapping pattern is continuously repeated the designated number of times.

As an example, the user may perform the tapping operation corresponding to 'tap-break-tap-tap-tap', which is the first tapping pattern. The processor 310 may identify the first tapping pattern based on data related to tapping according to the tapping operation of the user. The processor 310 may identify that the first tapping pattern is repeated three times. Based on identifying that the first tapping pattern is repeated three times, the processor 310 may identify that the user of the wearable device 200 is in the emergency state.

In operation 940, the processor 310 may transmit a first signal to an external electronic device to indicate that the user is in the emergency state.

For example, the processor 310 may provide a notification for identifying whether to transmit the first signal to indicate that the user is in the emergency state, based on identifying that the user is in the emergency state. The processor 310 may provide the notification using at least one component. As an example, the processor 310 may provide the notification for identifying whether to transmit the first signal by using at least one of sound including a siren and a beep, a vibration, and/or blinking of light emitting circuitry. According to an embodiment, the processor 310 may provide the notification for identifying whether to transmit the first signal through the external electronic device connected with the wearable device 200.

Based on the provision of the notification, the processor 310 may identify another tapping pattern for requesting transmission of the first signal. In response to identifying the other tapping pattern for requesting transmission of the first signal, the processor 310 may transmit the first signal for indicating that the user is in the emergency state to the external electronic device.

As an example, the processor 310 may identify the tapping pattern indicating whether the first signal is transmitted based on the provision of the notification. A second tapping pattern may indicate to transmit the first signal. A third tapping pattern may indicate not to transmit the first signal. The processor 310 may transmit the first signal for indicating that the user is in the emergency state to the external electronic device based on identifying the second tapping pattern.

According to an embodiment, the external electronic device may be configured for an emergency SOS service. For example, the external electronic device may include a server for performing the emergency SOS service. According to an embodiment, the external electronic device may include a device corresponding to a designated contact (e.g., an emergency contact). For example, the processor 310 may directly transmit the first signal to the external electronic device to indicate that the user is in the emergency state using communication circuitry (e.g., the communication circuitry 320 of FIG. 3A).

According to an embodiment, the external electronic device may include a device configured to transmit a second signal for an outgoing call to a device corresponding to one of a plurality of emergency contacts. The processor 310 may transmit the first signal to cause the external electronic device to transmit the second signal for the outgoing call to the device corresponding to the one of the plurality of emergency contacts. The processor 310 may transmit the first signal to the external electronic device. The external electronic device may receive the first signal. The external electronic device may transmit the second signal for the outgoing call to the device corresponding to the one of the plurality of emergency contacts based on the first signal.

According to an embodiment, the processor 310 may transmit the first signal to the external electronic device to indicate that the user is in the emergency state based on a designated time period. For example, the first signal may not be properly transmitted to the external electronic device. Accordingly, the processor 310 may transmit the first signal to the external electronic device based on the designated time period.

According to an embodiment, the external electronic device may include a satellite. The processor 310 may transmit the first signal to the external electronic device through satellite communication. The processor 310 may transmit the first signal to the external electronic device through the satellite communication based on the designated time period.

According to an embodiment, the processor 310 may transmit the first signal based on the designated time period by using a wireless Bluetooth low energy (BLE) disaster network. For example, the first signal may be broadcast. The processor 310 may indicate that the user of the wearable device 200 is in the emergency state by transmitting the first signal to external electronic devices within a designated distance from the wearable device 200.

According to an embodiment, the processor 310 may identify a position of the wearable device 200 based on identifying that the user is in the emergency state. The wearable device 200 may identify the position of the wearable device 200 using position identification circuitry (not illustrated). The processor 310 may activate the position identification circuitry based on identifying that the user is in the emergency state.

According to an embodiment, the processor 310 may execute (or perform) the designated function in the external electronic device based on identifying that the user is in the emergency state. For example, the processor 310 may transmit the first signal for executing a position tracking function of the external electronic device to the external electronic device based on identifying that the user is in the emergency state.

According to an embodiment, the processor 310 may transmit information (e.g., blood pressure information, body temperature information, and pulse information) on the user and/or information (e.g., battery information and position information) on the wearable device 200 through the first signal.

According to an embodiment, the processor 310 may identify a tapping input for an external object. In order to identify the tapping input for the external object, the processor 310 may identify that a distance between the wearable device 200 and the external object is within a designated distance. The processor 310 may identify the first tapping pattern while the distance between the wearable device 200 and the external object is within the designated distance. The processor 310 may transmit the first signal to the external electronic device based on identifying the first tapping pattern.

For example, the processor 310 may identify an input of tapping the external object according to the first tapping pattern as an input for an emergency SOS of the user. In order to identify the tapping input for the external object, the processor 310 may identify that the distance between the wearable device 200 and the external object is within the designated distance. The processor 310 may identify the first tapping pattern while the distance between the wearable device 200 and the external object is within the designated distance. The processor 310 may transmit the first signal for the emergency SOS to the external electronic device based on identifying the first tapping pattern.

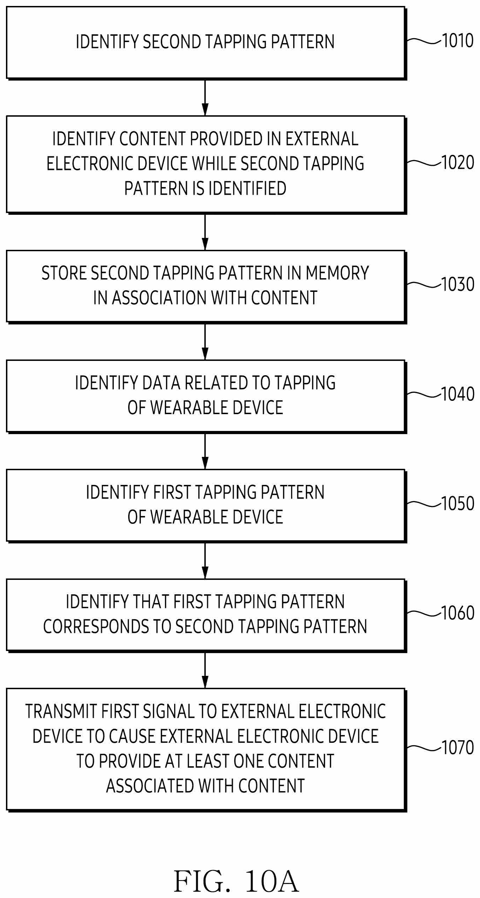

FIG. 10A illustrates a flowchart of an operation of a wearable device according to an embodiment. A wearable device 200 may perform operations illustrated in FIG. 10A. Hereinafter, the operations illustrated in FIG. 10A will be described as being performed by a processor (e.g., the processor 310 of FIG. 3A). In the following embodiment, each of the operations may be sequentially performed, but is not necessarily performed sequentially. For example, an order of each of the operations may be changed, and at least two operations may be performed in parallel.

Referring to FIG. 10A, in operation 1010, a processor 310 may identify a second tapping pattern. The processor 310 may identify the second tapping pattern based on data related to tapping of the wearable device 200.

In operation 1020, the processor 310 may identify a content provided in an external electronic device while the second tapping pattern is identified. The processor 310 may identify the content provided in the external electronic device in a state that a user wears the wearable device 200. For example, the content provided in the external electronic device may include music, a video, a multimedia content, and/or an application.

In operation 1030, the processor 310 may store the second tapping pattern in memory (e.g., the memory 340 of FIG. 3A) in association with the content. For example, the processor 310 may store the second tapping pattern in association with the content in the memory 340 by training a model (e.g., a neural network model) indicated by a plurality of parameters based on information (e.g., beats per minute (BPM) information and waveform information) on the content and the second tapping pattern.

For example, the processor 310 may identify that the second tapping pattern occurs while the content is provided. The processor 310 may identify that the second tapping pattern occurs while the content is provided at different times (or dates). For example, the processor 310 may repeatedly train the model (e.g., the neural network model) indicated by the plurality of parameters based on the information (e.g., the beats per minute (BPM) information and the waveform information) on the content and the second tapping pattern. As the model is repeatedly trained, accuracy of the model may increase.

In operation 1040, the processor 310 may identify the data related to the tapping. For example, the wearable device 200 may identify the data related to the tapping using a sensor (e.g., the sensor 330 of FIG. 3A) during a designated time interval. The operation 1040 may correspond to the operation 410 of FIG. 4.

In operation 1050, the processor 310 may identify a first tapping pattern of the wearable device 200. For example, the processor 310 may identify the first tapping pattern of the wearable device 200 based on the identified data. The operation 1050 may correspond to the operation 420 of FIG. 4.

In operation 1060, the processor 310 may identify that the first tapping pattern corresponds to the second tapping pattern. For example, the processor 310 may identify that the first tapping pattern corresponds to the second tapping pattern by setting the first tapping pattern as an input value of the model (e.g., the neural network model) indicated by the plurality of parameters.

For example, the processor 310 may identify a first impact pattern configuring the first tapping pattern. The processor 310 may identify a second impact pattern configuring the second tapping pattern. The processor 310 may identify that the first tapping pattern corresponds to the second tapping pattern based on a BPM information of the first impact pattern and a BPM information of the second impact pattern.

In operation 1070, the processor 310 may transmit a first signal to the external electronic device to cause the external electronic device to provide at least one content associated with the content. For example, in response to identifying that the first tapping pattern corresponds to the second tapping pattern, the processor 310 may transmit the first signal to the external electronic device to cause the external electronic device to provide the at least one content associated with the content.

For example, the processor 310 may provide the at least one content through the external electronic device in response to identifying that the first tapping pattern corresponds to the second tapping pattern. As an example, the at least one content may include the content. As an example, the at least one content may include a candidate content for providing to the user identified based on the content.

For example, the processor 310 may identify the second tapping pattern while a second music is being played in the external electronic device. The processor 310 may recommend at least one music related to the second music to the user based on identifying the first tapping pattern corresponding to the second tapping pattern. As an example, the at least one music may include the second music. The processor 310 may recommend the second music based on identifying the first tapping pattern corresponding to the second tapping pattern.

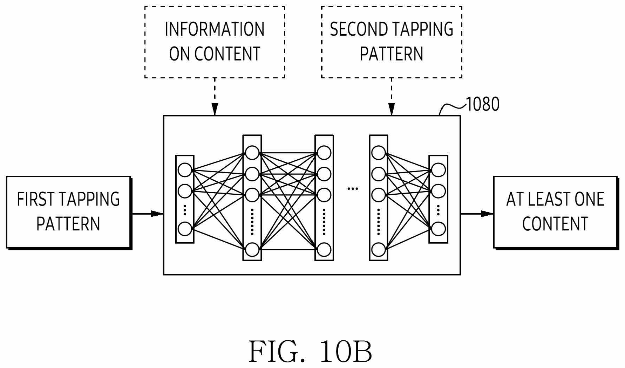

FIG. 10B illustrates an example of an operation of a wearable device according to an embodiment.

Referring to FIG. 10B, a processor 310 may train a model 1080 indicated by a plurality of parameters. The processor 310 may identify a second tapping pattern. The processor 310 may identify a content provided in an external electronic device while the second tapping pattern is identified.

The processor 310 may train the model 1080 based on information (e.g., BPM information or waveform information) on the content and the second tapping pattern. The processor 310 may repeatedly train the model 1080.

For example, the processor 310 may identify that music is provided through the external electronic device while the second tapping pattern is identified. The processor 310 may identify information on the music provided in the external electronic device. The information on the music provided in the external electronic device may include the BPM information, genre (e.g., classic, jazz, pop, ballad, rhythm and blues, hip-hop, and country music) information, singer information, title information, and/or mood information. The processor 310 may train the model 1080 based on the information on the music and the second tapping pattern.

The processor 310 may identify a first tapping pattern after the model 1080 is trained. The processor 310 may set the first tapping pattern as an input value of the model 1080. The processor 310 may identify at least one content based on an output value of the model 1080. The processor 310 may transmit a first signal to the external electronic device to cause the external electronic device to provide the at least one content.

For example, the processor 310 may identify at least one music based on setting the first tapping pattern as the input value of the model 1080. The processor 310 may identify at least one music associated with the music provided while the second tapping pattern is identified. As an example, a BPM value of the at least one music may be the same as and/or similar to a BPM value of the music provided while the second tapping pattern is identified. As an example, a genre of the at least one music may be the same as and/or similar to a genre of the music provided while the second tapping pattern is identified. As an example, a singer of the at least one music may be the same as a singer of the music provided while the second tapping pattern is identified. As an example, a mood of the at least one music may be the same as and/or similar to a mood of the music provided while the second tapping pattern is identified.

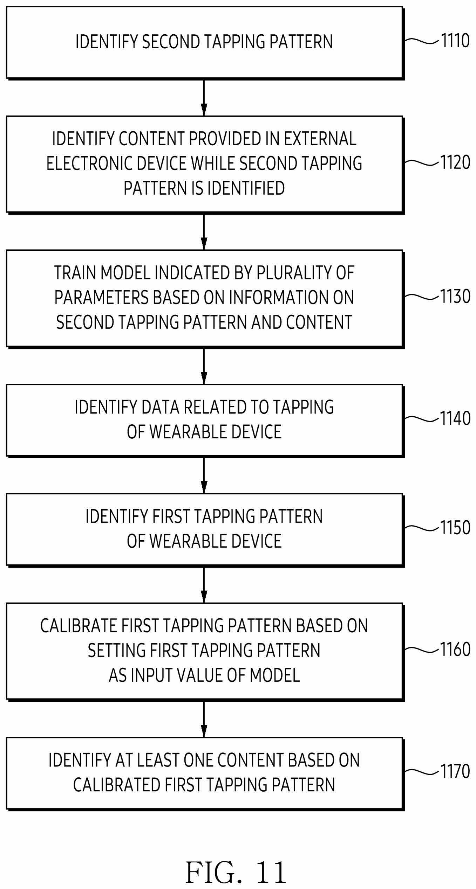

FIG. 11 illustrates a flowchart of an operation of a wearable device according to an embodiment. A wearable device 200 may perform operations illustrated in FIG. 11. Hereinafter, the operations illustrated in FIG. 11 will be described as being performed by a processor (e.g., the processor 310 of FIG. 3A). In the following embodiment, each of the operations may be sequentially performed, but is not necessarily performed sequentially. For example, an order of each of the operations may be changed, and at least two operations may be performed in parallel.

Referring to FIG. 11, in operation 1110, a processor 310 may identify a second tapping pattern. A processor 310 may identify the second tapping pattern based on data related to tapping. The operation 1110 may correspond to the operation 1010 of FIG. 10A.

In operation 1120, the processor 310 may identify a content (e.g., music) provided in an external electronic device while the second tapping pattern is identified. The operation 1120 may correspond to the operation 1020 of FIG. 10A.

In operation 1130, the processor 310 may train a model (e.g., the model 1080 of FIG. 10B) indicated by a plurality of parameters based on information on the second tapping pattern and the content. For example, the information on the content may include a BPM value of the content. The processor 310 may train the model indicated by the plurality of parameters based on the BPM value for the second tapping pattern and the BPM value of the content.

For example, the processor 310 may identify a difference between the BPM value for the second tapping pattern and the BPM value of the content. The processor 310 may train the model based on the difference between the BPM value for the second tapping pattern and the BPM value of the content.

As an example, the difference between the BPM value of the content (e.g., the music) and the BPM value for the second tapping pattern may occur due to a user's habit of riding a rhythm and movement of the user that are not identifiable in the wearable device 200. Accordingly, the processor 310 may train the model to calibrate the difference.

In operation 1140, the processor 310 may identify the data related to the tapping. For example, the wearable device 200 may identify the data related to the tapping using a sensor (e.g., the sensor 330 of FIG. 3A) during a designated time interval. The operation 1140 may correspond to the operation 410 of FIG. 4.

In operation 1150, the processor 310 may identify a first tapping pattern of the wearable device. For example, the processor 310 may identify the first tapping pattern of the wearable device 200 based on the identified data. The operation 1150 may correspond to the operation 420 of FIG. 4.

In operation 1160, the processor 310 may calibrate the first tapping pattern based on setting the first tapping pattern as an input value of the model indicated by the plurality of parameters.

According to an embodiment, the processor 310 may identify the calibrated value based on setting the first tapping pattern as the input value of the model. The processor 310 may calibrate the first tapping pattern based on the calibrated value.

For example, the processor 310 may identify a BPM value related to the first tapping pattern. For example, the BPM value related to the first tapping pattern may be identified based on the following equation. [Equation 2] $BPM = \frac{N}{T} \times 60$

Equation 2 above is merely an example for helping understanding, and is not limited thereto, and may be modified, applied, or extended in various methods.

Referring to Equation 2, the BPM is the BPM value for the first tapping pattern. The N is the number of tapping identified during a designated time. The T is the designated time (unit: seconds).

The processor 310 may set the BPM value related to the first tapping pattern as the input value of the model. The processor 310 may identify the calibrated value based on an output value of the model. The processor 310 may calibrate the BPM value related to the first tapping pattern based on the identified calibrated value. For example, the processor 310 may calibrate the BPM value related to the first tapping pattern based on the following equation. [Equation 3] $BPM _ A = BPM + α$

Equation 3 above is merely an example for helping understanding, and is not limited thereto, and may be modified, applied, or extended in various methods.

Referring to Equation 3, the BPM_A is the calibrated BPM value related to the first tapping pattern. The BPM is the BPM value related to the first tapping pattern. The α (alpha) is the calibrated value.

The processor 310 may calibrate the first tapping pattern based on calibrating the BPM value related to the first tapping pattern.

In operation 1170, the processor 310 may identify at least one content based on the calibrated first tapping pattern. For example, the processor 310 may identify the at least one content based on the BPM value (e.g., the BPM_A) related to the calibrated first tapping pattern. For example, the at least one content (e.g., the music) having the same and/or similar BPM value as the BPM value related to the calibrated first tapping pattern may be identified.

According to an embodiment, the processor 310 may identify history information on the content provided in the external electronic device. The processor 310 may identify a plurality of content based on the history information. The processor 310 may identify, among the plurality of content, the at least one content (e.g., the music) having the same and/or similar BPM value as the BPM value related to the calibrated first tapping pattern.

According to an embodiment, the processor 310 may identify the plurality of content stored in the external electronic device. The processor 310 may identify, among the plurality of content, the at least one content (e.g., the music) having the same and/or similar BPM value as the BPM value related to the calibrated first tapping pattern.

According to an embodiment, the processor 310 may identify the plurality of content set as favorites by the user. The processor 310 may identify, among the plurality of content, the at least one content (e.g., the music) having the same and/or similar BPM value as the BPM value related to the calibrated first tapping pattern.

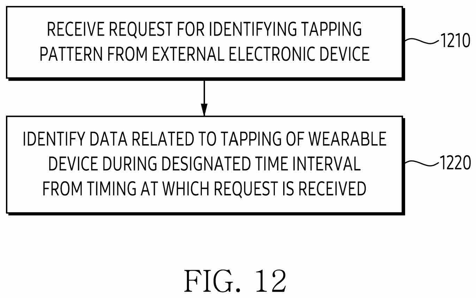

FIG. 12 illustrates a flowchart of an operation of a wearable device according to an embodiment. A wearable device 200 may perform operations illustrated in FIG. 12. Hereinafter, the operations illustrated in FIG. 12 will be described as being performed by a processor (e.g., the processor 310 of FIG. 3A). In the following embodiment, each of the operations may be sequentially performed, but is not necessarily performed sequentially. For example, an order of each of the operations may be changed, and at least two operations may be performed in parallel.

Referring to FIG. 12, in operation 1210, a processor 310 may receive a request for identifying a tapping pattern from an external electronic device.

In operation 1220, the processor 310 may identify data related to tapping of an electronic device during a designated time interval from a timing at which the request is received.

According to the operation 1210 and the operation 1220, a user of the wearable device 200 may request the external electronic device to perform a designated function. The external electronic device may provide a notification for confirming whether to perform the designated function. The user may perform a response to the notification through the tapping pattern of the wearable device 200.

According to an embodiment, the processor 310 may receive the request for identifying the tapping pattern from the external electronic device. The processor 310 may activate the sensor 330 (e.g., the acceleration sensor 331 of FIG. 3A) from the timing at which the request is received. The processor 310 may identify the data related to the tapping during the designated time interval from the timing at which the request is received. The processor 310 may identify the tapping pattern based on the data related to the tapping.

The processor 310 may identify whether to perform the designated function based on the tapping pattern. For example, the processor 310 may transmit a signal for indicating to perform the designated function to the external electronic device based on the first tapping pattern. For example, the processor 310 may transmit a signal for indicating not to perform the designated function to the external electronic device based on a second tapping pattern.

For example, the external electronic device may receive a voice input from the user to request that another external electronic device (e.g., a TV) be turned on. The external electronic device may provide the user with a notification for confirming whether to turn on power of the other external electronic device. The external electronic device may transmit, together with the notification, the request for identifying the tapping pattern to the wearable device 200. The processor 310 of the wearable device 200 may identify the tapping pattern based on the request. The processor 310 may transmit one of a signal for indicating to perform the designated function and a signal for indicating not to perform the designated function to the external electronic device based on the tapping pattern. The external electronic device may identify whether to turn on the power of the other external electronic device (e.g., the TV) based on the signal received from the wearable device 200.

For example, the external electronic device may be configured to perform a calling function. In a case that a call is received, the external electronic device may provide a notification. The external electronic device may transmit the request for identifying the tapping pattern together with the notification to the wearable device 200. The processor 310 of the wearable device 200 may identify the tapping pattern based on the request. The processor 310 may transmit one of the signal for indicating to perform the designated function and the signal for indicating not to perform the designated function to the external electronic device based on the tapping pattern. The external electronic device may identify whether to receive a call based on the signal received from the wearable device 200.

As in the above-described embodiment, the processor 310 may activate a sensor 330 (e.g., an acceleration sensor 331) and identify the tapping pattern during a designated time, based on the request received from the external electronic device. Since the tapping pattern is identified based on the request, the probability of malfunction may be reduced.

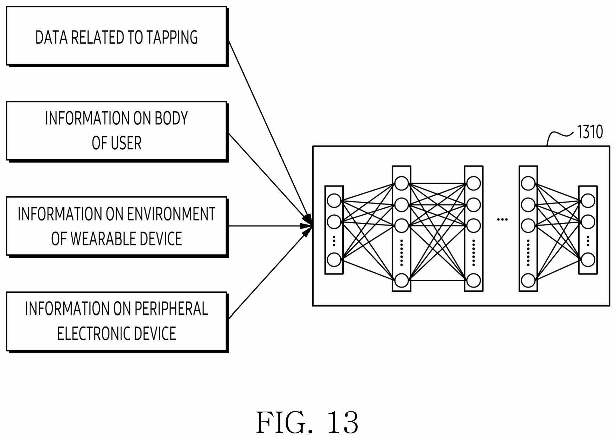

FIG. 13 illustrates an example of an operation of training a model in a wearable device according to an embodiment.

Referring to FIG. 13, a processor (e.g., the processor 310 of FIG. 3A) of a wearable device 200 may train a model 1310 indicated by a plurality of parameters. The model 1310 may correspond to the model 1080 of FIG. 10B.

According to an embodiment, the processor 310 may train the model 1310 based on various information. For example, the processor 310 may train the model 1310 based on at least one of data related to tapping of a user, information on a body of the user, information (e.g., a temperature, humidity, and brightness) on an environment of the wearable device 200, and/or information on a peripheral electronic device.

For example, the processor 310 may identify a plurality of tapping patterns based on at least one of the data related to the tapping of the user, the information on the body of the user, the information on the environment of the wearable device 200, and/or the information on the peripheral electronic device. As an example, the information on the body of the user may include at least one of body temperature information and/or heart rate information.

For example, the processor 310 may train the model 1310 based on the heart rate information of the user and information on a baseline of the body temperature of the user. In a case that the user is in an emergency situation, the heart rate and the body temperature may increase due to activation of a sympathetic nerve of the user. The processor 310 may set the information (e.g., the heart rate information and the body temperature information) on the body of the user and the data related to the tapping of the user as an input value of the model 1310. The processor 310 may identify a first tapping pattern as an output value of the model 1310. The processor 310 may identify a function related to the emergency situation corresponding to the first tapping pattern.

According to the above-described embodiment, the processor 310 may identify an accurate tapping pattern by training the model 1310 based on not only the data related to the tapping but also additional information (e.g., the information on the body of the user, the information on the environment of the wearable device 200, and the information on the peripheral electronic device). For example, the processor 310 may set the data related to the tapping and the additional information (e.g., the information on the body of the user, the information on the environment of the wearable device 200, and the information on the peripheral electronic device) as the input value of the model 1310. The processor 310 may identify the accurate tapping pattern based on the output value of the model 1310.

For example, the processor 310 may identify various states of the user based on the additional information (e.g., the information on the body of the user, the information on the environment of the wearable device 200, and the information on the peripheral electronic device). The processor 310 may identify the accurate tapping pattern in consideration of the various states of the user.

As an example, the processor 310 may identify that the user is in a driving state based on the additional information (e.g., the information on the body of the user, the information on the environment of the wearable device 200, and the information on the peripheral electronic device). As an example, the processor 310 may identify that the user is in a state of listening to music based on the additional information (e.g., the information on the body of the user, the information on the environment of the wearable device 200, and the information on the peripheral electronic device). As an example, the processor 310 may identify that the user is in a state of exercising based on the additional information (e.g., the information on the body of the user, the information on the environment of the wearable device 200, and the information on the peripheral electronic device).

According to an embodiment, the processor 310 may identify tapping pattern sets that are set differently according to a situation. The processor 310 may identify one of a plurality of situations based on at least one of the information on the body of the user, the information on the environment of the wearable device 200, and/or the information on the peripheral electronic device. The processor 310 may identify a tapping pattern set corresponding to the one of the plurality of situations. The processor 310 may identify a plurality of tapping patterns included in the tapping pattern set. For example, the processor 310 may identify the first tapping pattern among the plurality of tapping patterns based on data related to an orientation of the wearable device 200 and data related to a change in acceleration of the wearable device 200.

As an example, the processor 310 may set the information on the body of the user, the information on the environment of the wearable device 200, and/or the information on the peripheral electronic device as the input value of the model 1310. The processor 310 may identify the tapping pattern set as the output value of the model 1310. The processor 310 may identify a plurality of tapping patterns included in the tapping pattern set. The processor 310 may identify the first tapping pattern among the plurality of tapping patterns based on the data related to the tapping. The processor 310 may identify a function corresponding to the first tapping pattern.

The above-described model 1310 may be configured with one model or a combination of various models. For example, in a case that the model 1310 is configured with the one model, the model 1310 may output different types of output values according to a type of the input value. For example, in a case that the model 1310 is configured with a combination of a plurality of models, the model 1310 may output an output value through one of the plurality of models, according to the type of the input value. Accordingly, the processor 310 may obtain various types of output values based on inputting various types of input values to the model 1310. Hereinafter, an example of the output value according to the input value will be described.

For example, the processor 310 may set the data related to the tapping of the wearable device 200 as the input value of the model 1310. The processor 310 may identify the tapping pattern based on the output value of the model 1310.

For example, the processor 310 may set the tapping pattern as the input value of the model 1310. The processor 310 may identify at least one content based on the output value of the model 1310.

For example, the processor 310 may set the first tapping pattern as the input value of the model 1310. Based on the output value of the model 1310, the processor 310 may identify that the first tapping pattern corresponds to a second tapping pattern stored in memory (e.g., the memory 340 of FIG. 3A).

For example, the processor 310 may set the first tapping pattern as the input value of the model 1310. The processor 310 may calibrate the first tapping pattern based on the output value of the model 1310.

For example, the processor 310 may set a BPM value related to the first tapping pattern as the input value of the model 1310. The processor 310 may identify the calibrated value based on the output value of the model 1310. The processor 310 may calibrate the BPM value related to the first tapping pattern based on the identified calibrated value.

According to an embodiment, a wearable device (e.g., the wearable device 290) may include a ring-shape housing (e.g., the housing 210) including a first surface facing a part of a body of a user and a second surface opposite to the first surface, communication circuitry (e.g., the communication circuitry 320) disposed between the first surface and the second surface, one or more sensors (e.g., the sensor 330) disposed between the first surface and the second surface, at least one processor (e.g., the processor 310) disposed between the first surface and the second surface, and including process circuitry, and memory (e.g., the memory 340), including one or more storage media, storing instructions. The instructions, when executed by the at least one processor individually or collectively, may cause the wearable device to identify, using the one or more sensors, data related to tapping of the wearable device during a designated time interval. The instructions, when executed by the at least one processor individually or collectively, may cause the wearable device to identify, based on the data, a first tapping pattern of the wearable device. The instructions, when executed by the at least one processor individually or collectively, may cause the wearable device to, based on identifying a function corresponding to the first tapping pattern, transmit a first signal related to the function to an external electronic device connected with the wearable device.

According to an embodiment, the instructions, when executed by the at least one processor individually or collectively, may cause the wearable device to, based on identifying the first tapping pattern continuously repeated a designated number of times, identify that the user of the wearable device is in an emergency state. The instructions, when executed by the at least one processor individually or collectively, may cause the wearable device to transmit the first signal to the external electronic device to indicate that the user is in the emergency state.

According to an embodiment, the external electronic device may be configured to transmit a second signal for an outgoing call to a device corresponding to one of a plurality of emergency contacts. The instructions, when executed by the at least one processor individually or collectively, may cause the wearable device to transmit the first signal to cause the external electronic device to transmit the second signal for the outgoing call to the device corresponding to the one of the plurality of emergency contacts.

According to an embodiment, the instructions, when executed by the at least one processor individually or collectively, may cause the wearable device to, based on identifying that the user is in the emergency state, provide a notification to identify whether to transmit the first signal to indicate that the user is in the emergency state. The instructions, when executed by the at least one processor individually or collectively, may cause the wearable device to, based on the providing of the notification, in response to identifying another tapping pattern for requesting transmission of the first signal, transmit the first signal to the external electronic device to indicate that the user is in the emergency state.

According to an embodiment, the instructions, when executed by the at least one processor individually or collectively, may cause the wearable device to transmit, based on a designated time period, the first signal to the external electronic device to indicate that the user is in the emergency state.

According to an embodiment, the external electronic device may be configured for an emergency SOS service.

According to an embodiment, the instructions, when executed by the at least one processor individually or collectively, may cause the wearable device to identify, using the one or more sensors, information on a body of the user. The instructions, when executed by the at least one processor individually or collectively, may cause the wearable device to identify, based on the information on the body of the user, that a state of the user is a designated state. The instructions, when executed by the at least one processor individually or collectively, may cause the wearable device to identify the function corresponding to the first tapping pattern identified based on the data while the state of the user is the designated state.

According to an embodiment, the instructions, when executed by the at least one processor individually or collectively, may cause the wearable device to identify that a distance between the wearable device and an external object is within a designated distance. The instructions, when executed by the at least one processor individually or collectively, may cause the wearable device to transmit, based on identifying the first tapping pattern, the first signal to the external electronic device while the distance between the wearable device and the external object is within the designated distance.

According to an embodiment, the instructions, when executed by the at least one processor individually or collectively, may cause the wearable device to receive a request for identifying a tapping pattern from the external electronic device. The instructions, when executed by the at least one processor individually or collectively, may cause the wearable device to, in response to the request, identify, using the one or more sensors, the data related to the tapping during the designated time interval from a timing at which the request is received.

According to an embodiment, the instructions, when executed by the at least one processor individually or collectively, may cause the wearable device to identify a second tapping pattern. The instructions, when executed by the at least one processor individually or collectively, may cause the wearable device to identify a content provided in the external electronic device during identifying the second tapping pattern. The instructions, when executed by the at least one processor individually or collectively, may cause the wearable device to store the second tapping pattern in the memory in association with the content.

According to an embodiment, the instructions, when executed by the at least one processor individually or collectively, may cause the wearable device to identify that the first tapping pattern identified based on the data corresponds to the second tapping pattern. The instructions, when executed by the at least one processor individually or collectively, may cause the wearable device to, in response to identifying that the first tapping pattern corresponds to the second tapping pattern, transmit the first signal to cause the external electronic device to provide at least one content related to the content, to the external electronic device.

According to an embodiment, the instructions, when executed by the at least one processor individually or collectively, may be set to cause the wearable device to train a model indicated by a plurality of parameters based on the second tapping pattern and information on the content. The instructions, when executed by the at least one processor individually or collectively, may cause the wearable device to calibrate the first tapping pattern based on setting the first tapping pattern as an input value of the model. The instructions, when executed by the at least one processor individually or collectively, may cause the wearable device to, based on the calibrated first tapping pattern, identify the at least one content.

According to an embodiment, the at least one content may include a candidate content for providing to the user, which is identified based on the content.

According to an embodiment, the data on the tapping may include data on an orientation of the wearable device and data on an acceleration change of the wearable device. The instructions, when executed by the at least one processor individually or collectively, may cause the wearable device to identify the first tapping pattern among a plurality of tapping patterns based on the data on the orientation of the wearable device and the data on the acceleration change of the wearable device.

According to an embodiment, the instructions, when executed by the at least one processor individually or collectively, may cause the wearable device to identify the plurality of tapping patterns based on at least one of the information on the body of the user, information on an environment of the wearable device, and information on a peripheral electronic device.

According to an embodiment, the designated time interval may be one of a plurality of time intervals set based on a sliding window scheme.

According to an embodiment, the first tapping pattern may be identified based on tapping intensity and a tapping period identified in the wearable device.

According to an embodiment, a method of a wearable device (e.g., the wearable device 200) may include identifying, using one or more sensors (e.g., the sensor 330) of the wearable device, data related to tapping during a designated time interval. The method may include identifying, based on the data, a first tapping pattern of the wearable device. The method may include, based on identifying a function corresponding to the first tapping pattern, transmitting a first signal related to the function to an external electronic device connected with the wearable device 290.

According to an embodiment, the method may include, based on identifying the first tapping pattern continuously repeated a designated number of times, identifying that the user of the wearable device 290 is in an emergency state. The method may include transmitting the first signal to the external electronic device to indicate that the user is in the emergency state.

According to an embodiment, the external electronic device may be configured to transmit a second signal for an outgoing call to a device corresponding to one of a plurality of emergency contacts. The method may include transmitting the first signal to cause the external electronic device to transmit the second signal for the outgoing call to the device corresponding to the one of the plurality of emergency contacts.

According to an embodiment, the method may include, based on identifying that the user is in the emergency state, providing a notification to identify whether to transmit the first signal to indicate that the user is in the emergency state. The method may include, based on the providing of the notification, in response to identifying another tapping pattern for requesting transmission of the first signal, transmitting the first signal to the external electronic device to indicate that the user is in the emergency state.

According to an embodiment, a non-transitory computer readable storage medium may store one or more programs. The one or more programs may include instructions which, when executed by at least one processor (e.g., the processor 310) of a wearable device (e.g., the wearable device 290) with one or more sensors (e.g., the sensor 330) and communication circuitry (e.g., the communication circuitry 320), cause the wearable device to identify, using the one or more sensors 330, data related to tapping of the wearable device during a designated time interval. The one or more programs may include instructions which, when executed by the at least one processor, cause the wearable device to identify, based on the data, a first tapping pattern of the wearable device 200. The one or more programs may include instructions which, when executed by the at least one processor, cause the wearable device to, based on identifying a function corresponding to the first tapping pattern, transmit a first signal related to the function to an external electronic device connected with the wearable device.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between a case in which data is semi-permanently stored in the storage medium and a case in which the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable device (200) comprising:
a ring-shaped housing (210) comprising a first surface contacting a part of a body of a user when the wearable device (200) is worn and a second surface disposed opposite to the first surface;
communication circuitry (320);
one or more sensors (330);
at least one processor (310) comprising processing circuitry; and
memory (340), comprising one or more storage media storing instructions,
wherein the instructions, when executed by the at least one processor (310) individually or collectively, cause the wearable device (200) to:
obtain, via the one or more sensors (330), motion data of the wearable device (200) and orientation data of the wearable device (200), during a designated time interval,
based on the motion data being determined as a defined tapping gesture of a hand of the user wearing the wearable device (200) while the orientation data is determined as corresponding to a first orientation, transmit, via the communication circuitry (320), a first signal causing an external electronic device to perform a first function, and
based on the motion data being determined as the defined tapping gesture while the orientation data is determined as corresponding to a second orientation distinct from the first orientation, transmit, via the communication circuitry (320), a second signal causing the external electronic device to perform a second function distinct from the first function.

2. The wearable device (200) of claim 1, wherein the instructions, when executed by the at least one processor (310) individually or collectively, cause the wearable device (200) to:
based on identifying that the defined tapping gesture is repeated a designated number of times, identify that the user of the wearable device (200) is in an emergency state, and
transmit, to the external electronic device, a signal to indicate that the user is in the emergency state.

3. The wearable device (200) of claim 2, wherein the external electronic device is configured to transmit a signal for an outgoing call to a device corresponding to one of a plurality of emergency contacts, and
wherein the instructions, when executed by the at least one processor (310) individually or collectively, cause the wearable device (200) to transmit a command for causing the external electronic device to transmit the signal for the outgoing call to the device corresponding to the one of the plurality of emergency contacts.

4. The wearable device (200) of claim 2, wherein the instructions, when executed by the at least one processor (310) individually or collectively, cause the wearable device (200) to:
based on identifying that the user is in the emergency state, provide a notification to identify whether to transmit the signal to indicate that the user is in the emergency state, and
based on the notification, in response to identifying another tapping gesture for requesting transmission of the signal to indicate that the user is in the emergency state, transmit, to the external electronic device, the signal to indicate that the user is in the emergency state.

5. The wearable device (200) of claim 2, wherein the instructions, when executed by the at least one processor (310) individually or collectively, cause the wearable device (200) to transmit, periodically based on a designated time period, the signal to indicate that the user is in the emergency state to the external electronic device.

6. The wearable device (200) of claim 2, wherein the signal indicating that the user is in the emergency state includes biometric information on the user and location information of the wearable device, and
wherein the signal is configured to cause the external electronic device, which provides an emergency SOS service, to execute a position tracking function of the external electronic device.

7. The wearable device (200) of claim 1, wherein the instructions, when executed by the at least one processor (310) individually or collectively, cause the wearable device (200) to:
identify, using the one or more sensors (330), biometric information on the user,
identify, based on the biometric information on the user, that the user is in an emergency state, and
identify a function corresponding to the defined tapping gesture identified based on the motion data while the user is in the emergency state.

8. The wearable device (200) of claim 1, wherein the instructions, when executed by the at least one processor (310) individually or collectively, cause the wearable device (200) to:
identify that a distance between the wearable device (200) and an external object is within a designated distance, and
while the distance between the wearable device (200) and the external object is within the designated distance, based on identifying that the defined tapping gesture is performed on the external object, transmit a signal to the external electronic device.

9. The wearable device (200) of claim 1, wherein the instructions, when executed by the at least one processor (310) individually or collectively, cause the wearable device (200) to:
receive a request for identifying a tapping gesture from the external electronic device, and
in response to the request, obtain, using the one or more sensors (330), the motion data during the designated time interval from a timing at which the request is received.

10. The wearable device (200) of claim 1, wherein the instructions, when executed by the at least one processor (310) individually or collectively, cause the wearable device (200) to:
identify another tapping gesture,
identify a content provided in the external electronic device while the another tapping gesture is identified, and
store the another tapping gesture in the memory (340) in association with the content.

11. The wearable device (200) of claim 10, wherein the instructions, when executed by the at least one processor (310) individually or collectively, cause the wearable device (200) to:
identify that the defined tapping gesture identified based on the motion data corresponds to the another tapping gesture, and
in response to identifying that the defined tapping gesture corresponds to the another tapping gesture, transmit, to the external electronic device, a signal for causing the external electronic device to provide at least one content related to the content.

12. The wearable device (200) of claim 11, wherein the instructions, when executed by the at least one processor (310) individually or collectively, cause the wearable device (200) to:
train a model indicated by a plurality of parameters based on the another tapping gesture and information on the content,
calibrate the defined tapping gesture by inputting the defined tapping gesture into the model, and
based on the calibrated tapping gesture, identify the at least one content.

13. The wearable device (200) of claim 11, wherein the at least one content comprises a recommended content for the user, related to the content.

14. A method of operating a wearable device (200) comprising a ring-shaped housing (210) comprising a first surface contacting a part of a body of a user when the wearable device (200) is worn and a second surface disposed opposite to the first surface, communication circuitry (320), and one or more sensors (330), comprising:
obtaining, via the one or more sensors (330), motion data of the wearable device (200) and orientation data of the wearable device (200), during a designated time interval,
based on the motion data being determined as a defined tapping gesture of a hand of a user wearing the wearable device (200) while the orientation data is determined as corresponding to a first orientation, transmitting, via the communication circuitry (320), a first signal causing an external electronic device to perform a first function, and
based on the motion data being determined as the defined tapping gesture while the orientation data is determined as corresponding to a second orientation distinct from the first orientation, transmitting, via the communication circuitry (320), a second signal causing the external electronic device to perform a second function distinct from the first function.

15. A non-transitory computer readable storage medium storing one or more programs, the one or more programs comprise instructions which, when executed by at least one processor (310) of a wearable device (200) comprising a ring-shaped housing (210) comprising a first surface contacting a part of a body of a user when the wearable device (200) is worn and a second surface disposed opposite to the first surface, communication circuitry (320), and one or more sensors (330), cause the wearable device (200) to:
obtain, via the one or more sensors (330), motion data of the wearable device (200) and orientation data of the wearable device (200), during a designated time interval,
based on the motion data being determined as a defined tapping gesture of a hand of a user wearing the wearable device (200) while the orientation data is determined as corresponding to a first orientation, transmit, via the communication circuitry (320), a first signal causing an external electronic device to perform a first function, and
based on the motion data being determined as the defined tapping gesture while the orientation data is determined as corresponding to a second orientation distinct from the first orientation, transmit, via the communication circuitry (320), a second signal causing the external electronic device to perform a second function distinct from the first function.

## Patentansprüche

1. Wearable-Vorrichtung (200), umfassend:
ein ringförmiges Gehäuse (210), das eine erste Oberfläche, die einen Teil eines Körpers eines Benutzers berührt, wenn die Wearable-Vorrichtung (200) getragen wird, und eine zweite Oberfläche, die gegenüber der ersten Oberfläche angeordnet ist, umfasst;
eine Kommunikationsschaltung (320);
einen oder mehrere Sensoren (330);
mindestens einen Prozessor (310), der eine Verarbeitungsschaltung umfasst; und
Speicher (340), der ein oder mehrere Speichermedien umfasst, die Anweisungen speichern,
wobei die Anweisungen, wenn sie durch den mindestens einen Prozessor (310) einzeln oder kollektiv ausgeführt werden, die Wearable-Vorrichtung (200) dazu veranlassen:
Bewegungsdaten der Wearable-Vorrichtung (200) und Orientierungsdaten der Wearable-Vorrichtung (200) über den einen oder die mehreren Sensoren (330) während eines vorgegebenen Zeitintervalls zu erlangen,
basierend darauf, dass die Bewegungsdaten als eine definierte Tippgeste einer Hand des Benutzers bestimmt werden, der die Wearable-Vorrichtung (200) trägt, während die Orientierungsdaten als einer ersten Orientierung entsprechend bestimmt werden, ein erstes Signal über die Kommunikationsschaltung (320) zu übertragen, das eine externe elektronische Vorrichtung dazu veranlasst, eine erste Funktion durchzuführen, und
basierend darauf, dass die Bewegungsdaten als die definierte Tippgeste bestimmt werden, während die Orientierungsdaten als einer zweiten Orientierung entsprechend bestimmt werden, die sich von der ersten Orientierung unterscheidet, ein zweites Signal über die Kommunikationsschaltung (320) zu übertragen, das die externe elektronische Vorrichtung dazu veranlasst, eine zweite Funktion durchzuführen, die sich von der ersten Funktion unterscheidet.

2. Wearable-Vorrichtung (200) nach Anspruch 1, wobei die Anweisungen, wenn sie durch den mindestens einen Prozessor (310) einzeln oder kollektiv ausgeführt werden, die Wearable-Vorrichtung (200) dazu veranlassen:
basierend darauf, dass identifiziert wird, dass die definierte Tippgeste eine vorgegebene Anzahl von Malen wiederholt wird, zu identifizieren, dass sich der Benutzer der Wearable-Vorrichtung (200) in einem Notfallzustand befindet, und
ein Signal, um anzugeben, dass sich der Benutzer in dem Notfallzustand befindet, an die externe elektronische Vorrichtung zu übertragen.

3. Wearable-Vorrichtung (200) nach Anspruch 2, wobei die externe elektronische Vorrichtung dazu konfiguriert ist, ein Signal für einen ausgehenden Anruf an eine Vorrichtung zu übertragen, die einem einer Vielzahl von Notfallkontakten entspricht, und
wobei die Anweisungen, wenn sie durch den mindestens einen Prozessor (310) einzeln oder kollektiv ausgeführt werden, die Wearable-Vorrichtung (200) dazu zu veranlassen, einen Befehl zum Veranlassen zu übertragen, dass die externe elektronische Vorrichtung das Signal für den ausgehenden Anruf an die Vorrichtung überträgt, die dem einen der Vielzahl von Notfallkontakten entspricht.

4. Wearable-Vorrichtung (200) nach Anspruch 2, wobei die Anweisungen, wenn sie durch den mindestens einen Prozessor (310) einzeln oder kollektiv ausgeführt werden, die Wearable-Vorrichtung (200) dazu veranlassen:
basierend darauf, dass identifiziert wird, dass sich der Benutzer in dem Notfallzustand befindet, eine Benachrichtigung bereitzustellen, um zu identifizieren, ob das Signal, um anzugeben, dass sich der Benutzer in dem Notfallzustand befindet, zu übertragen ist, und
basierend auf der Benachrichtigung als Reaktion darauf, dass eine andere Tippgeste zum Anfordern einer Übertragung des Signals, um anzugeben, dass sich der Benutzer in dem Notfallzustand befindet, identifiziert wird, das Signal, um anzugeben, dass sich der Benutzer in dem Notfallzustand befindet, an die externe elektronische Vorrichtung zu übertragen.

5. Wearable-Vorrichtung (200) nach Anspruch 2, wobei die Anweisungen, wenn sie durch den mindestens einen Prozessor (310) einzeln oder kollektiv ausgeführt werden, die Wearable-Vorrichtung (200) dazu zu veranlassen, das Signal, um anzugeben, dass sich der Benutzer in dem Notfallzustand befindet, periodisch basierend auf einem vorgegebenen Zeitraum an die externe elektronische Vorrichtung zu übertragen.

6. Wearable-Vorrichtung (200) nach Anspruch 2, wobei das Signal, das angibt, dass sich der Benutzer in dem Notfallzustand befindet, biometrische Informationen über den Benutzer und Standortinformationen der Wearable-Vorrichtung einschließt und
wobei das Signal dazu konfiguriert ist, die externe elektronische Vorrichtung, die einen Notfall-SOS-Dienst bereitstellt, dazu zu veranlassen, eine Positionsverfolgungsfunktion der externen elektronischen Vorrichtung auszuführen.

7. Wearable-Vorrichtung (200) nach Anspruch 1, wobei die Anweisungen, wenn sie durch den mindestens einen Prozessor (310) einzeln oder kollektiv ausgeführt werden, die Wearable-Vorrichtung (200) dazu veranlassen:
unter Verwendung des einen oder der mehreren Sensoren (330) biometrische Informationen über den Benutzer zu identifizieren,
basierend auf den biometrischen Informationen über den Benutzer zu identifizieren, dass sich der Benutzer in einem Notfallzustand befindet, und
eine Funktion zu identifizieren, die der definierten Tippgeste entspricht, die basierend auf den Bewegungsdaten identifiziert wird, während sich der Benutzer in dem Notfallzustand befindet.

8. Wearable-Vorrichtung (200) nach Anspruch 1, wobei die Anweisungen, wenn sie durch den mindestens einen Prozessor (310) einzeln oder kollektiv ausgeführt werden, die Wearable-Vorrichtung (200) dazu veranlassen:
zu identifizieren, dass eine Entfernung zwischen der Wearable-Vorrichtung (200) und einem externen Objekt innerhalb einer vorgegebenen Entfernung liegt, und
während die Entfernung zwischen der Wearable-Vorrichtung (200) und dem externen Objekt innerhalb der vorgegebenen Entfernung liegt, basierend darauf, dass identifiziert wird, dass die definierte Tippgeste an dem externen Objekt durchgeführt wird, ein Signal an die externe elektronische Vorrichtung zu übertragen.

9. Wearable-Vorrichtung (200) nach Anspruch 1, wobei die Anweisungen, wenn sie durch den mindestens einen Prozessor (310) einzeln oder kollektiv ausgeführt werden, die Wearable-Vorrichtung (200) dazu veranlassen:
eine Anforderung eines Identifizierens einer Tippgeste von der externen elektronischen Vorrichtung zu empfangen und
als Reaktion auf die Anforderung unter Verwendung des einen oder der mehreren Sensoren (330) die Bewegungsdaten während des vorgegebenen Zeitintervalls ab einem Zeitpunkt zu erlangen, zu dem die Anforderung empfangen wird.

10. Wearable-Vorrichtung (200) nach Anspruch 1, wobei die Anweisungen, wenn sie durch den mindestens einen Prozessor (310) einzeln oder kollektiv ausgeführt werden, die Wearable-Vorrichtung (200) dazu veranlassen:
eine andere Tippgeste zu identifizieren,
einen Inhalt, der in der externen elektronischen Vorrichtung bereitgestellt wird, zu identifizieren, während die andere Tippgeste identifiziert wird, und
die andere Tippgeste in Assoziation mit dem Inhalt in dem Speicher (340) zu speichern.

11. Wearable-Vorrichtung (200) nach Anspruch 10, wobei die Anweisungen, wenn sie durch den mindestens einen Prozessor (310) einzeln oder kollektiv ausgeführt werden, die Wearable-Vorrichtung (200) dazu veranlassen:
zu identifizieren, dass die definierte Tippgeste, die basierend auf den Bewegungsdaten identifiziert wird, der anderen Tippgeste entspricht, und
als Reaktion darauf, dass identifiziert wird, dass die definierte Tippgeste der anderen Tippgeste entspricht, ein Signal zum Veranlassen, dass die externe elektronische Vorrichtung mindestens einen Inhalt bereitstellt, der sich auf den Inhalt bezieht, an die externe elektronische Vorrichtung zu übertragen.

12. Wearable-Vorrichtung (200) nach Anspruch 11, wobei die Anweisungen, wenn sie durch den mindestens einen Prozessor (310) einzeln oder kollektiv ausgeführt werden, die Wearable-Vorrichtung (200) dazu veranlassen:
ein Modell, das durch eine Vielzahl von Parametern angegeben ist, basierend auf der anderen Tippgeste und Informationen über den Inhalt zu trainieren,
die definierte Tippgeste durch Eingeben der definierten Tippgeste in das Modell zu kalibrieren und
basierend auf der kalibrierten Tippgeste den mindestens einen Inhalt zu identifizieren.

13. Wearable-Vorrichtung (200) nach Anspruch 11, wobei der mindestens eine Inhalt einen empfohlenen Inhalt für den Benutzer umfasst, der sich auf den Inhalt bezieht.

14. Verfahren zum Betreiben einer Wearable-Vorrichtung (200), umfassend ein ringförmiges Gehäuse (210), das eine erste Oberfläche, die einen Teil eines Körpers eines Benutzers berührt, wenn die Wearable-Vorrichtung (200) getragen wird, und eine zweite Oberfläche, die gegenüber der ersten Oberfläche angeordnet ist, umfasst, eine Kommunikationsschaltung (320) und einen oder mehrere Sensoren (330), umfassend:
Erlangen von Bewegungsdaten der Wearable-Vorrichtung (200) und Orientierungsdaten der Wearable-Vorrichtung (200) über den einen oder die mehreren Sensoren (330) während eines vorgegebenen Zeitintervalls,
basierend darauf, dass die Bewegungsdaten als eine definierte Tippgeste einer Hand eines Benutzers bestimmt werden, der die Wearable-Vorrichtung (200) trägt, während die Orientierungsdaten als einer ersten Orientierung entsprechend bestimmt werden, Übertragen eines ersten Signals über die Kommunikationsschaltung (320), das eine externe elektronische Vorrichtung dazu veranlasst, eine erste Funktion durchzuführen, und
basierend darauf, dass die Bewegungsdaten als die definierte Tippgeste bestimmt werden, während die Orientierungsdaten als einer zweiten Orientierung entsprechend bestimmt werden, die sich von der ersten Orientierung unterscheidet, Übertragen eines zweiten Signals über die Kommunikationsschaltung (320), das die externe elektronische Vorrichtung dazu veranlasst, eine zweite Funktion durchzuführen, die sich von der ersten Funktion unterscheidet.

15. Nichttransitorisches computerlesbares Speichermedium, das ein oder mehrere Programme speichert, das eine oder die mehreren Programme umfassen Anweisungen, die, wenn sie durch mindestens einen Prozessor (310) einer Wearable-Vorrichtung (200) ausgeführt werden, umfassend ein ringförmiges Gehäuse (210), das eine erste Oberfläche, die einen Teil eines Körpers eines Benutzers berührt, wenn die Wearable-Vorrichtung (200) getragen wird, und eine zweite Oberfläche, die gegenüber der ersten Oberfläche angeordnet ist, umfasst, eine Kommunikationsschaltung (320) und einen oder mehrere Sensoren (330), die Wearable-Vorrichtung (200) dazu veranlassen:
Bewegungsdaten der Wearable-Vorrichtung (200) und Orientierungsdaten der Wearable-Vorrichtung (200) über den einen oder die mehreren Sensoren (330) während eines vorgegebenen Zeitintervalls zu erlangen,
basierend darauf, dass die Bewegungsdaten als eine definierte Tippgeste einer Hand eines Benutzers bestimmt werden, der die Wearable-Vorrichtung (200) trägt, während die Orientierungsdaten als einer ersten Orientierung entsprechend bestimmt werden, ein erstes Signal über die Kommunikationsschaltung (320) zu übertragen, das eine externe elektronische Vorrichtung dazu veranlasst, eine erste Funktion durchzuführen, und
basierend darauf, dass die Bewegungsdaten als die definierte Tippgeste bestimmt werden, während die Orientierungsdaten als einer zweiten Orientierung entsprechend bestimmt werden, die sich von der ersten Orientierung unterscheidet, ein zweites Signal über die Kommunikationsschaltung (320) zu übertragen, das die externe elektronische Vorrichtung dazu veranlasst, eine zweite Funktion durchzuführen, die sich von der ersten Funktion unterscheidet.

## Revendications

1. Dispositif pouvant être porté (200), comprenant :
un boîtier en forme d'anneau (210) comprenant une première surface en contact avec une partie du corps d'un utilisateur lorsque le dispositif pouvant être porté (200) est porté et une seconde surface disposée à l'opposé de la première surface ;
des circuits de communication (320) ;
un ou plusieurs capteurs (330) ;
au moins un processeur (310) comprenant des circuits de traitement ; et
une mémoire (340) comprenant un ou plusieurs supports de stockage stockant des instructions ;
dans lequel les instructions, lorsqu'elles sont exécutées par l'au moins un processeur (310) individuellement ou collectivement, amènent le dispositif pouvant être porté (200) à :
obtenir, via l'un ou plusieurs capteurs (330), des données de mouvement du dispositif pouvant être porté (200) et des données d'orientation du dispositif pouvant être porté (200), pendant un intervalle de temps désigné,
sur la base de la détermination des données de mouvement comme un geste de tapotement défini d'une main de l'utilisateur portant le dispositif pouvant être porté (200) alors que les données d'orientation sont déterminées comme correspondant à une première orientation, transmettre, via les circuits de communication (320), un premier signal provoquant l'exécution d'une première fonction par un dispositif électronique externe, et
sur la base de la détermination des données de mouvement comme le geste de tapotement défini alors que les données d'orientation sont déterminées comme correspondant à une deuxième orientation distincte de la première orientation, transmettre, via les circuits de communication (320), un deuxième signal provoquant l'exécution d'une deuxième fonction distincte de la première fonction par le dispositif électronique externe.

2. Dispositif pouvant être porté (200) selon la revendication 1, dans lequel les instructions, lorsqu'elles sont exécutées par l'au moins un processeur (310) individuellement ou collectivement, amènent le dispositif pouvant être porté (200) à :
sur la base de l'identification que le geste de tapotement défini est répété un nombre désigné de fois, identifier que l'utilisateur du dispositif pouvant être porté (200) est dans un état d'urgence, et
transmettre, au dispositif électronique externe, un signal pour indiquer que l'utilisateur est en état d'urgence.

3. Dispositif pouvant être porté (200) selon la revendication 2, dans lequel le dispositif électronique externe est configuré pour transmettre un signal d'appel sortant à un dispositif correspondant à l'un d'une pluralité de contacts d'urgence, et
dans lequel les instructions, lorsqu'elles sont exécutées par l'au moins un processeur (310) individuellement ou collectivement, amènent le dispositif pouvant être porté (200) à transmettre une commande pour amener le dispositif électronique externe à transmettre le signal d'appel sortant au dispositif correspondant à l'un de la pluralité de contacts d'urgence.

4. Dispositif pouvant être porté (200) selon la revendication 2, dans lequel les instructions, lorsqu'elles sont exécutées par l'au moins un processeur (310) individuellement ou collectivement, amènent le dispositif pouvant être porté (200) à :
sur la base de l'identification que l'utilisateur est en état d'urgence, fournir une notification pour identifier s'il faut transmettre le signal pour indiquer que l'utilisateur est en état d'urgence, et
sur la base de la notification, en réponse à l'identification d'un autre geste de tapotement demandant la transmission du signal pour indiquer que l'utilisateur est en état d'urgence, transmettre, au dispositif électronique externe, le signal pour indiquer que l'utilisateur est en état d'urgence.

5. Dispositif pouvant être porté (200) selon la revendication 2, dans lequel les instructions, lorsqu'elles sont exécutées par l'au moins un processeur (310) individuellement ou collectivement, amènent le dispositif pouvant être porté (200) à transmettre, périodiquement sur la base d'une période de temps désignée, le signal pour indiquer que l'utilisateur est dans l'état d'urgence au dispositif électronique externe.

6. Dispositif pouvant être porté (200) selon la revendication 2, dans lequel le signal indiquant que l'utilisateur est dans l'état d'urgence comprend des informations biométriques sur l'utilisateur et des informations de localisation du dispositif pouvant être porté, et
dans lequel le signal est configuré pour amener le dispositif électronique externe, qui fournit un service SOS d'urgence, à exécuter une fonction de suivi de position du dispositif électronique externe.

7. Dispositif pouvant être porté (200) selon la revendication 1, dans lequel les instructions, lorsqu'elles sont exécutées par l'au moins un processeur (310) individuellement ou collectivement, amènent le dispositif pouvant être porté (200) à :
identifier, à l'aide de l'un ou plusieurs capteurs (330), des informations biométriques sur l'utilisateur,
identifier, sur la base des informations biométriques sur l'utilisateur, que l'utilisateur est dans un état d'urgence, et
identifier une fonction correspondant au geste de tapotement défini identifié sur la base des données de mouvement pendant que l'utilisateur est dans l'état d'urgence.

8. Dispositif pouvant être porté (200) selon la revendication 1, dans lequel les instructions, lorsqu'elles sont exécutées par l'au moins un processeur (310) individuellement ou collectivement, amènent le dispositif pouvant être porté (200) à :
identifier que la distance entre le dispositif pouvant être porté (200) et un objet externe est inférieure ou égale à une distance désignée, et
tandis que la distance entre le dispositif pouvant être porté (200) et l'objet externe est inférieure ou égale à la distance désignée, sur la base de l'identification que le geste de tapotement défini est effectué sur l'objet externe, transmettre un signal au dispositif électronique externe.

9. Dispositif pouvant être porté (200) selon la revendication 1, dans lequel les instructions, lorsqu'elles sont exécutées par l'au moins un processeur (310) individuellement ou collectivement, amènent le dispositif pouvant être porté (200) à :
recevoir une demande d'identification d'un geste de tapotement de la part du dispositif électronique externe, et
en réponse à la demande, obtenir, à l'aide de l'un ou plusieurs capteurs (330), les données de mouvement pendant l'intervalle de temps désigné à partir d'un moment auquel la demande est reçue.

10. Dispositif pouvant être porté (200) selon la revendication 1, dans lequel les instructions, lorsqu'elles sont exécutées par l'au moins un processeur (310) individuellement ou collectivement, amènent le dispositif pouvant être porté (200) à :
identifier un autre geste de tapotement,
identifier un contenu fourni dans le dispositif électronique externe pendant que l'autre geste de tapotement est identifié, et
stocker l'autre geste de tapotement dans la mémoire (340) en association avec le contenu.

11. Dispositif pouvant être porté (200) selon la revendication 10, dans lequel les instructions, lorsqu'elles sont exécutées par l'au moins un processeur (310) individuellement ou collectivement, amènent le dispositif pouvant être porté (200) à :
identifier que le geste de tapotement défini identifié sur la base des données de mouvement correspond à l'autre geste de tapotement, et
en réponse à l'identification que le geste de tapotement défini correspond à l'autre geste de tapotement, transmettre, au dispositif électronique externe, un signal pour amener le dispositif électronique externe à fournir au moins un contenu lié au contenu.

12. Dispositif pouvant être porté (200) selon la revendication 11, dans lequel les instructions, lorsqu'elles sont exécutées par l'au moins un processeur (310) individuellement ou collectivement, amènent le dispositif pouvant être porté (200) à :
entraîner un modèle indiqué par une pluralité de paramètres sur la base de l'autre geste de tapotement et des informations sur le contenu,
calibrer le geste de tapotement défini en entrant le geste de tapotement défini dans le modèle, et
sur la base du geste de tapotement calibré, identifier l'au moins un contenu.

13. Dispositif pouvant être porté (200) selon la revendication 11, dans lequel l'au moins un contenu comprend un contenu recommandé pour l'utilisateur, lié au contenu.

14. Procédé de fonctionnement d'un dispositif pouvant être porté (200) comprenant un boîtier en forme d'anneau (210) comprenant une première surface en contact avec une partie du corps d'un utilisateur lorsque le dispositif pouvant être porté (200) est porté et une seconde surface disposée à l'opposé de la première surface, des circuits de communication (320) et un ou plusieurs capteurs (330), comprenant :
l'obtention, via l'un ou plusieurs capteurs (330), de données de mouvement du dispositif pouvant être porté (200) et de données d'orientation du dispositif pouvant être porté (200), pendant un intervalle de temps désigné,
sur la base de la détermination des données de mouvement comme un geste de tapotement défini d'une main d'un utilisateur portant le dispositif pouvant être porté (200) alors que les données d'orientation sont déterminées comme correspondant à une première orientation, la transmission, via les circuits de communication (320), d'un premier signal provoquant l'exécution d'une première fonction par un dispositif électronique externe, et
sur la base de la détermination des données de mouvement comme le geste de tapotement défini alors que les données d'orientation sont déterminées comme correspondant à une deuxième orientation distincte de la première orientation, la transmission, via les circuits de communication (320), d'un deuxième signal provoquant l'exécution d'une deuxième fonction distincte de la première fonction par le dispositif électronique externe.

15. Support de stockage lisible par ordinateur non transitoire stockant un ou plusieurs programmes, l'un ou plusieurs programmes comprennent des instructions qui, lorsqu'elles sont exécutées par au moins un processeur (310) d'un dispositif pouvant être porté (200) comprenant un boîtier en forme d'anneau (210) comprenant une première surface en contact avec une partie du corps d'un utilisateur lorsque le dispositif pouvant être porté (200) est porté et une seconde surface disposée à l'opposé de la première surface, des circuits de communication (320) et un ou plusieurs capteurs (330), amènent le dispositif pouvant être porté (200) à :
obtenir, via l'un ou plusieurs capteurs (330), des données de mouvement du dispositif pouvant être porté (200) et des données d'orientation du dispositif pouvant être porté (200), pendant un intervalle de temps désigné,
sur la base de la détermination des données de mouvement comme un geste de tapotement défini d'une main de l'utilisateur portant le dispositif pouvant être porté (200) alors que les données d'orientation sont déterminées comme correspondant à une première orientation, transmettre, via les circuits de communication (320), un premier signal provoquant l'exécution d'une première fonction par un dispositif électronique externe, et
sur la base de la détermination des données de mouvement comme le geste de tapotement défini alors que les données d'orientation sont déterminées comme correspondant à une deuxième orientation distincte de la première orientation, transmettre, via les circuits de communication (320), un deuxième signal provoquant l'exécution d'une deuxième fonction distincte de la première fonction par le dispositif électronique externe.
